(19) **Europäisches Patentamt / European Patent Office / Office européen des brevets**

(11) **EP 1 410 776 B1**

(12) **EUROPEAN PATENT SPECIFICATION**

(45) Date of publication and mention
of the grant of the patent:
**18.02.2009 Bulletin 2009/08**

(21) Application number: **02771752.9**

(22) Date of filing: **21.05.2002**

(51) Int Cl.:
*A61F 13/15* (2006.01)      *A61F 13/472* (2006.01)
*A61F 13/534* (2006.01)      *A61F 13/539* (2006.01)

(86) International application number:
**PCT/JP2002/004892**

(87) International publication number:
**WO 2002/094156 (28.11.2002 Gazette 2002/48)**

(54) **INTERLABIAL PAD**

INTERLABIAL-POLSTER

SERVIETTE INTERLABIALE

(84) Designated Contracting States:
**AT BE CH CY DE DK ES FI FR GB GR IE IT LI LU MC NL PT SE TR**

(30) Priority: **22.05.2001   JP 2001152403**
                **06.08.2001   JP 2001238511**

(43) Date of publication of application:
**21.04.2004   Bulletin 2004/17**

(73) Proprietor: **UNI-CHARM CORPORATION**
**Shikokuchuo-shi,**
**Ehime 799-0111 (JP)**

(72) Inventors:
 • **MIZUTANI, Satoshi**
   **Technical Center, Uni-Charm Corp**
   **Mitoyo-gun,**
   **Kagawa 769-1602 (JP)**
 • **YAMAKI, Koichi**
   **Technical Center, Uni-Charm Corp.**
   **Mitoyo-gun,**
   **Kagawa 769-1602 (JP)**

 • **NODA, Yuki,**
   **Technical Center, Uni-Charm Corp.**
   **Mitoyo-gun,**
   **Kagawa 769-1602 (JP)**

(74) Representative: **Stark, Gordon Drummond et al**
**Murgitroyd & Company**
**Scotland House**
**165-169 Scotland Street**
**Glasgow**
**G5 8PL (GB)**

(56) References cited:
**EP-A- 0 815 816          EP-A- 0 956 843**
**EP-A2- 1 097 685         WO-A1-94/17115**
**WO-A1-98/08475           WO-A1-98/57610**
**WO-A1-99/01093           WO-A1-99/01096**
**WO-A1-99/26575           WO-A1-99/55272**
**JP-A- 10 033 589**

 • **PATENT ABSTRACTS OF JAPAN vol. 1999, no. 09, 30 July 1999 (1999-07-30) & JP 11 104168 A (KAO CORP), 20 April 1999 (1999-04-20)**

**Description**

**Background of the Invention**

**Technical Field**

The present invention relates to an interlabial pad with a high prevention effect of menstrual blood leak, which can be easily fitted between female labia.

**Background Art**

Conventionally, a sanitary napkin and a tampon are used generally as sanitary products for female. Here, there have been great efforts made to prevent the leak of menstrual blood from gap caused by poor adhesion near the ostium vaginae as for the sanitary napkin. Moreover, as for the tampon, there have been great efforts made for relieving the foreign feeling and the discomfort when wearing the sanitary products and intervaginal fixing trouble due to the nature of those products.

Under such situation, sanitary products of the interlabial pad have attracted people as a sanitary product positioned between the sanitary napkin and the tampon in recent years.

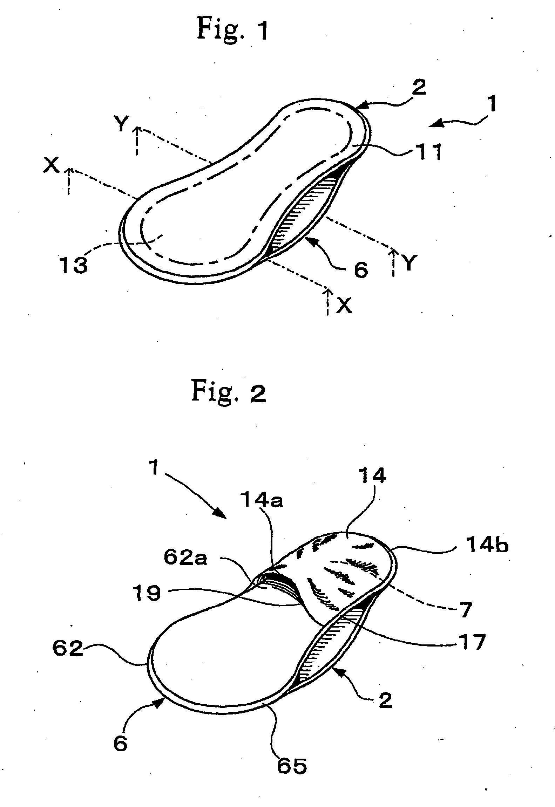

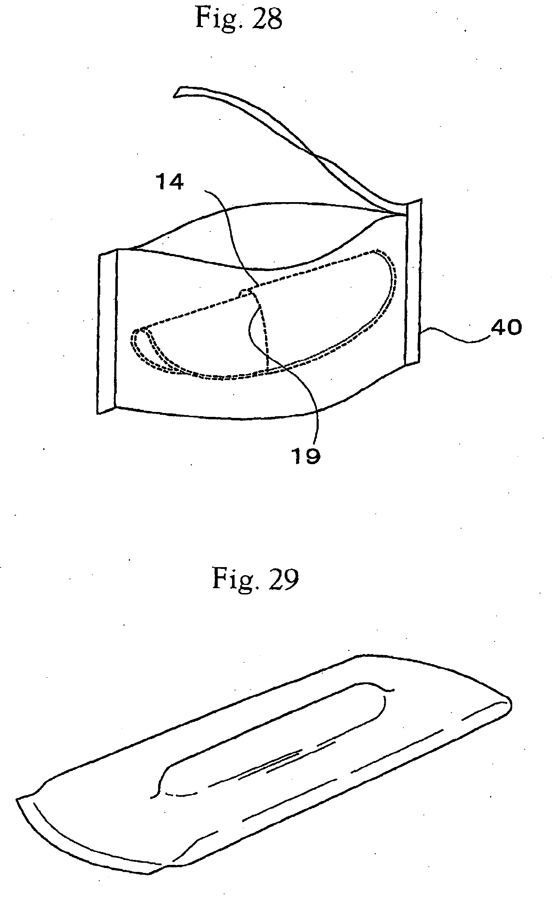

On the other hand, Japan Utility Model Laid Open Hei 5-18523 (see Fig. 29, which corresponds to Fig. 1 in this Specification) discloses a sanitary napkin in which an area projected from other area is provided by collectively providing an absorber in the central portion of the surface which comes to be in contact with the body of a wearer as the product improving the adhesion between the sanitary napkin and the body. In the configuration of the sanitary napkin, the projected area is inserted inside the labia so that the adhesion is improved compared to an ordinal sanitary napkin.

However, in the napkin as described, it is difficult to bring the projected area of the sanitary napkin to a predetermined position. In other words, in the improved sanitary napkin as described above, it is necessary, in regards to the sanitary napkin fixed to the underwear, to well-lead the projected area of the sanitary napkin to the predetermined position between the labia by the action of wearing an underwear although a sanitary napkin is fixed to an underwear and fitted to a body by bringing the fixed napkin to the body together with the underwear. In this action, leading of the projected area of the sanitary napkin to the predetermined position is an indirect action so that the control becomes difficult and the wearing position becomes less precise. Also, the projected area is formed by increasing the thickness of the absorber, which may cause a strong foreign feeling to the wearer depending on the thickness.

On the contrary, the above-described interlabial pad of the present invention can obtain the same adhesion as that of the related art on the basis of the nature of its product, which is to insert the product in between the labia, without providing the projected area as in the example of the related art.

However, the interlabial pad is not easy to wear and position shift is likely to occur at the time of wearing since it is to be fitted between the labia which is hard to be viewed. Also, even if it is fitted in the appropriate position at the time of wearing, it may be shifted due to the activities of a wearer. When there is a position shift generated, the damage due to the menstrual blood leak will be enormous since the size of the pad is smaller than a sanitary napkin.

The present invention has been designed to overcome the foregoing problems. An object of the present invention is to provide an interlabial pad with a structure capable of sure and sanitary wearing, which can decrease menstrual blood leak.

Summary of the Invention

To overcome the forgoing problems, the present invention is characterized in that an interlabial pad has a dual structure and unbonded areas are provided in the right and left side edges. Thereby, each sheet body is deformed separately to some extent so that the problems can be suppressed even if there is a position shift generated during or after wearing.

Also, the interlabial pad has a structure which can be inserted by properly using the ball of finger tip which has a keen sense. Specifically, it is characterized in that a pocket to which a finger can be smoothly inserted is formed in the pad on the opposite side to the body side so that wearing can be smoothly achieved while inserting a finger thereto.

More specifically, the present invention provides the followings.

(1) An interlabial pad having a size capable of being easily inserted between female labia, comprising: a pair of absorbing sheet bodies for absorbing body fluid, wherein each of the absorbing sheet bodies is unbonded at right and left side edges in respect of a longitudinal direction while bonded at both ends in the longitudinal direction. The interlabial pad of the present invention has a dual structure for absorbing body fluid so that menstrual blood which is not absorbed in the sheet body on the side facing the body may be absorbed in the sheet body on the side facing the garment. Therefore, it has a higher prevention effect of menstrual blood leak compared to an interlabial pad having a single structure using the same amount of absorber.

Also, both sheet bodies are joined in the peripheral edges on both ends in the longitudinal direction, and the other areas, that is, the inner from the inner edge in the right and left side edges are unbonded. Therefore, both sheet bodies can be separated vertically and, even if one of the sheet body is deformed, the deformation is not directly transmitted to the other sheet body. Thereby, both sheet bodies can be deformed to different shapes.

(2) An interlabial pad according to (1), wherein said pair of absorbing sheet bodies comprising: a main sheet body comprising: a first permeable surface side sheet facing a body side; a first permeable or impermeable back side sheet facing an opposite side to the body side; and a first absorber for absorbing body fluid; wherein the first permeable surface side sheet and the first back side sheet are bonded each other to enclose the first absorber, and a sub-sheet body comprising: a second permeable surface side sheet facing a body side; a second permeable or impermeable back side sheet facing a garment side; and a second absorber for absorbing body fluid; wherein the second permeable surface side sheet and the second back side sheet are bonded each other to enclose the second absorber.

The interlabial pad of the present invention includes a main sheet body and a sub-sheet body. The body side surfaces of both sheet bodies are both permeable and the absorber is enclosed inside each sheet body. Therefore, menstrual blood can be absorbed in both sheet bodies.

If an impermeable material is used for the back side sheet of the main sheet body, it becomes possible to prevent the menstrual blood, which is permeated and absorbed in the sub-sheet body, from returning to the main sheet body by a pressure such as body pressure thereby enabling a decrease in discomfort due to dampness when wearing.

(3) An interlabial pad according to (1) or (2), wherein the unbonded areas of said main sheet body and said sub-sheet body in the right and left side edges are line-symmetrical with respect to a center line in the longitudinal direction of the interlabial pad.

In the interlabial pad of the present invention, the unbonded area of the main sheet body and the sub-sheet body is provided in the right and left side edges in line-symmetrical with respect to the center line in the longitudinal direction. Hence, within the range corresponding to the unbonded area, each sheet body can be deformed independently in the lateral direction of the interlabial pad. Therefore, when the interlabial pad does not fit in an appropriate position at the time of wearing or when a position shift is generated due to the activities of the wearer after wearing, the main sheet body is deformed to a convex shape to be inserted between the labia and directly absorbing menstrual blood. The sub-sheet body stays in a substantial flat shape and is positioned to cover the pudenda without completely conforming to the deformation of the main sheet body, thereby absorbing menstrual blood which is not absorbed in the main sheet body. As described, in the present invention, even in the case where the interlabial pad is fitted with its center line shifted from the center line of the labia, menstrual blood leak in the lateral direction can be effectively prevented.

In this respect, in the case where a conventional projection is provided extendedly in the longitudinal direction, if the interlabial pad does not fit in the appropriate position at the time of wearing, specifically, if there is a position shift of the interlabial pad in the lateral direction with respect to the body of a wearer, even a part of the projection is not inserted in the groove between the labia, which extends in the same direction as that of the interlabial pad.

On the contrary, in the interlabial pad according to the present invention, the main sheet body alone can be deformed into an appropriate shape capable of being inserted in the groove between the labia of the wearer. Therefore, there is no risk of generating a gap between the labia of the wearer and the interlabial pad so that menstrual blood leak can be effectively prevented. In addition, the sub-sheet body is not inserted between the labia thereby giving no strong foreign feeling to the wearer.

(4) An interlabial pad according to any one of (1) to (3), wherein said unbonded areas, in each of right and left side edges in respect of the longitudinal direction of the interlabial pad, are formed in a range of 2/5 to 4/5 of a whole portion of each side edge.

In the interlabial pad according to the present invention, the range of 2/5 to 4/5 of the whole portion of each side edges is left unbonded. If the unbonded area on the side area of the interlabial pad is shorter than "2/5 of the whole portion of each side edges on both right and left hand sides", the side area of the main sheet body is fixed, thereby limiting the versatility of possible form features of the main sheet body itself. Therefore, the main sheet body is hard to be deformed into a convex shape sufficient to be inserted between the labia. As a result, the contact area ratio between the main sheet body and the labia reduces so that the risk of falling the product and of leaking menstrual blood increases.

On the other hand, if the unbonded area in the side area of the interlabial pad is longer than "4/5 of the whole portion of each side edges on both right and left hand sides", the degree of contact between the main sheet body and the sub-sheet body decreases inevitably. Thus, due to the friction generated between the opposite side face to the body side face of the fitted interlabial pad and an underwear or napkin, there may be cases that the main sheet body and the sub-sheet body may be separated.

In this respect, in the interlabial pad according to the present invention, the unbonded area in the each side edge on right and left hand sides is provided with an appropriate range so that the aforementioned problems do not happen, and the pad can be appropriately inserted between the labia of the wearer. It is preferable that the unbonded area be provided within the area of 2/5 from the front end to 1/5 from the back end. By positioning the unbonded area as described, the wearing state can be provided more preferable.

Also, in the interlabial pad according to the present invention, the range in which the main sheet body and the sub-sheet body are bonded is provided widely at the front end compared to the back end. This is achieved in the light of the characteristic that menstrual blood and membrane have high affinity. That is, menstrual blood from the ostium vagina runs towards the front (near the clitoris) with membrane so that the leak is likely to occur in the front. Therefore, the bonding area between the sub-sheet body, which does not deform, and the main sheet body is widely formed as a substantial flat area. Thereby, the area can cover the pudenda enabling an interruption of the menstrual blood flow.

In this Specification, "front end" of the interlabial pad means the front of the wearer at the time of wearing the pad, that is, one of the end positioned near the clitoris, and "back end" means the back of the wearer at the time of wearing the pad, that is, the other end positioned near the anus.

(5) An interlabial pad according to any one of (1) to (4), wherein the length of said main sheet body in the lateral direction is equal to that of said sub-sheet body in the lateral direction or shorter than that of said sub-sheet body in the lateral direction.

In the interlabial pad according to the embodiment, the main sheet body is never longer than the sub-sheet body in the lateral direction. Therefore, menstrual blood which cannot be absorbed in the main sheet body can be absorbed in the sub-sheet body.

As for the main sheet body, if an impermeable material is used for the back side sheet, its length in the lateral direction is preferably the minimum length for covering the absorber. Thereby, menstrual blood which permeates the surface side sheet is interrupted by the back side sheet, which enables to prevent the case where the menstrual blood is not absorbed in the sub-sheet body.

(6) An interlabial pad according to any one of (1) to (5), wherein a stiffness of said main sheet body in the lateral direction is smaller than that of said sub-sheet body.

In the interlabial pad according to the present invention, there is a difference in the stiffness of the main sheet body from the sub-sheet body in the lateral direction. The stiffness of the main sheet body is made smaller than that of the sub-sheet body. Thus, when the interlabial pad is worn, although the main sheet body is deformed by the pinching force of the labia, the sub-sheet body stays in a substantial flat shape. Concretely, in the non-bonding area of the main sheet body and the sub-sheet body, the main sheet body is easily deformed toward the body side into a convex shape to fit between the interlabia, while the sub-sheet body maintains a substantial flat shape. Therefore, the sub-sheet body in a substantial flat shape is positioned to cover the pudenda so that the sub-sheet body stands perpendicular to the flowing direction of menstrual blood, which is not absorbed and flown from the main sheet body, thereby, the menstrual blood flown from the lateral direction is prevented from leaking.

(7) An interlabial pad according to (6), wherein the stiffness of said main sheet body in the lateral direction is not more than 1.5 mN.

In the interlabial pad according to the present invention, the stiffness in the center area of the main sheet body in the lateral direction is 1.5 mN or less. If the stiffness is larger than 1.5 mN, it is hard to be deformed into convex so that it becomes hard to surely fit the interlabial pad between the labia, and there may be cases that interlabial pad falls off. Even if the main sheet body is inserted without problems, it may deform the original shape of the labia of the wearer into a different shape, thereby giving a foreign feeling to the wearer.

In this respect, when the stiffness is set to be 1.5 mN or less as in the interlabial pad according to the present invention, only the main sheet body can be easily bent to be a convex shape without deforming the natural shape of labia of the wearer at the time of wearing thereof. Thereby, preferable and sure wearing of the interlabial pad can be achieved.

(8) An interlabial pad according to any one of (1) to (7), wherein said first absorber inside said main sheet body has a line for inducing bending in the center of the lateral directions.

In the interlabial pad according to the present invention, in the center area of the first absorber in the lateral direction in the main sheet, at least a streak of a bending induction line for forming a convex is extendedly provided in the longitudinal direction continuously or intermittently. Thereby, the main sheet body can be easily bent along the bending induction line and the projected area in a convex shape is quickly formed on the main sheet body.

The "bending induction line for forming a convex" can be formed by providing a difference in the stiffness between the area where the line is to be provided and the other area. For example, the area where the induction line is to be provided is compressed so that there is a difference in the density from that of the periphery, or in the step of laminating the absorber, the amount of lamination on both sides are more increased than that on the area where the induction lines are applied. As a result, a difference in the thickness thereof is provided. Also, a cut may be provided in the area where the induction line is to be applied.

(9) An interlabial pad according to any one of (1) to (8), wherein said sub-sheet body includes a protruded area, which is protruded towards the body side.

In the interlabial pad according to the embodiment, in the sub-sheet body, a protruded area protruding towards the body side is provided. Therefore, a wearer can apply a finger tip with a keen sense inside the protruded area from the opposite side to the body side. As a result, it becomes easy to detect the ostium vagina via the interlabial pad. In order to be able to fit the interlabial pad in the most suitable area, that is, the area where the center line of the interlabial pad coincides with the center line of the labia, it is preferable that the protruded area be provided in the center area in the lateral direction.

Incidentally, the protruded area, as long as it functions as described, may be provided continuously or may be partially provided in the area where the finger comes into contact.

(10) An interlabial pad according to any one of (1) to (9), wherein said sub-sheet body further includes a mini sheet piece for forming a finger insertion opening between said second back side sheet and the mini sheet piece on the garment side of the second back face side sheet of said sub-sheet body.

In the interlabial pad according to the present invention, at least one of sleeve ends of a mini sheet piece, which is attached on the opposite side face to the body side face of the interlabial pad, is not bonded to the surface of the back side sheet in the lateral direction of the back side sheet. Thus, there is a sleeve portion formed between the one of the sleeve ends of the mini sheet piece and the back side sheet, which are unbonded. The sleeve portion serves as a finger insertion opening (refer to Fig. 2) to which a finger can be inserted.

In the back side sheet in the longitudinal direction, the mini sheet piece is bonded only on the right and left sides of the back side sheet and the inside is unbonded (not pasted). Therefore, the mini sheet piece is attached over the area from one side to the other side of the back side sheet. In the area from one side to the other side, an opening (finger insertion space) to which a finger can be inserted for holding is formed.

In the lateral direction of the back side sheet, if both of the sleeve portions of the mini sheet body are unbonded to the back side sheet, the space for inserting a finger becomes a through hole (a state of a tunnel) and, when one of the sleeve portion of the mini sheet piece is bonded, the opening for inserting finger becomes a cave state.

In the present invention, "side area" of the back side sheet in the longitudinal direction include not only the area corresponding to the peripheral edge of the interlabial pad but also the periphery of the peripheral edge where the mini sheet piece can be bonded.

In the interlabial pad according to the present invention as described, the main sheet body is attached to the back side sheet so as to form the finger insertion opening and the gap for finger insert continued thereto. Therefore, by inserting a finger to the finger insertion opening, the interlabial pad can be temporarily fixed to the tip of a finger. Thereby, although wearing the pad is worn between the labia, which is a place hard to view, the interlabial pad can be inserted to an appropriate position while accurately detecting the precise wearing position.

(11) An interlabial pad according to any one of (1) to (10), wherein the interlabial pad is used together with a sanitary napkin.

With the interlabial pad according to the present invention as described, even though the pad is used together with a sanitary napkin, there is not much bad influence on wear feeling and also rash and stuffiness can be decreased.

In other words, there are some sanitary napkin users who use several pieces of napkins overlapped together when there is a large quantity of menstrual blood. However, there cause problems that it feels uncomfortable such as stiffness and affects the external appearance. Also, the overlapped sanitary napkins are put on one after another at the area not near the ostium of vagina where there is no need for the overlapped napkins, which causes rash and stuffiness.

In this respect, with the present invention, the sanitary product is overlapped only on the labia and its periphery so that there is no influence on the wear feeling and the external appearance. Also, rash and stuffiness in the breech and its periphery can be decreased.

Furthermore, when changing the pad, it becomes possible to change only the interlabial pad according to the present invention without changing the sanitary napkin. Therefore, there is no need for the wearer to carry around the sanitary napkins which are large enough to be noticed. The sanitary napkins herein are not only the napkins which are sold for absorbing menstrual blood but also include absorption sheets for vaginal discharge.

(12) An interlabial pad according to any one of (1) to (11), wherein said interlabial pad is a pad for an incontinence of urine.

According to the interlabial pad of the present invention, the pad can be used for absorbing incontinence of urine. That is ostium vaginae where the menstrual blood is discharged and a pee hole where urine is discharged locate between labia, and the interlabial pad of the present invention used between labia can absorb urine also.

As described hereinbefore, the pad of the present invention can absorb urine around labia, especially around the pee hole and is useful for the absorbing pad for incontinence of urine, especially for a light incontinence of urine.

(13) An interlabial pad according to any one of (1) to (11), wherein said interlabial pad is a pad for absorbing vaginal discharge.

In accordance with the present invention, the interlabial pad can be used for the pad for absorbing the vaginal discharge. That is the interlabial pad is used between labia and can absorb the excretion other than the menstrual blood from ostium vaginae for the use therefore (for absorbing the vaginal discharge).

As described above, the pad can absorb the vaginal discharge in order to decrease the discomfort for the user, and is useful for the user who is not menstruating.

(14) A wrapping body comprising: an interlabial pad according to any one of (1) to (13); and a wrapping container for individually wrapping the interlabial pad; wherein the interlabial pad is enclosed in the wrapping container.

In the present invention, the interlabial pad is wrapped individually so that a user can carry the pad one by one for usage. Hence, compared to the case where a plurality of the interlabial pads are wrapped in the same wrapping container, cleaner and more convenient handling can be achieved.

(15) A wrapping body comprising: an interlabial pad according to any one of (10) to (13); and a wrapping container for individually wrapping the interlabial pad, wherein said interlabial pad is enclosed in said wrapping container such that the interlabial pad is orthogonal to the wrapping container.

In the interlabial pad according to the present invention, the interlabial pad is enclosed anisotropically in the wrapping container. Therefore, the wrapped body is formed so that the finger insertion opening formed by the mini sheet piece is to be opened toward the wearer when opening the wrapping container. Therefore, the opening direction and the finger insert direction can be made in the same direction for the wearer so that the wearer can insert the finger easily.

Brief Description of the Drawings

Fig. 1 is a schematic perspective view showing the body side of an interlabial pad according to the embodiment;
Fig. 2 is a schematic perspective view showing the opposite side to the body of the interlabial pad according to the embodiment;
Fig. 3 is a cross section of the interlabial pad according to the embodiment taken along the line X-X shown in Fig. 1;
Fig. 4 is a cross section of the interlabial pad according to the embodiment taken along the line Y-Y in Fig. 1;
Fig. 5 is an explanatory illustration for describing the ratio of the bonded and unbonded area between the main sheet body and the sub-sheet body in the interlabial pad according to the embodiment;
Fig. 6 is an illustration showing the interlabial pad according to the embodiment to which a plurality of mini sheet pieces are attached;
Fig. 7 is an explanatory illustration for describing the whole girth inside the finger insertion opening of the mini sheet piece attached to the interlabial pad according to the embodiment;
Fig. 8 is an illustration showing the state in which the mini sheet piece attached to the interlabial pad according to the embodiment has the length of 10 % or more in the longitudinal direction;
Fig. 9 is an illustration showing the position of unbonded area in the back side sheet of the mini sheet piece attached to the interlabial pad according to the embodiment;
Fig. 10 is an explanatory illustration for describing the attachment position of the mini-sheet on the interlabial pad according to the embodiment;
Fig. 11 is an illustration showing the state in which dimension of the back side sheet of the main sheet is smaller than that of the surface side sheet of the sub-sheet body in the interlabial pad according to the embodiment;
Fig. 12 is an explanatory illustration for describing the state of inserting a forefinger to a finger insertion opening when using the interlabial pad according to the embodiment;
Fig. 13 is an illustration showing the state of wearing the interlabial pad according to the embodiment between the labia;
Fig.14 is a front cross section view showing the state of the mini sheet piece after wearing the interlabial pad according to the embodiment;
Fig. 15 is an explanatory illustration showing the state when removing the mini sheet piece by pulling it from the interlabial pad according to the embodiment;
Fig. 16 is a longitudinal cross section showing the state in which a bending induction line is applied to an absorber included inside the main sheet body of the interlabial pad according to the embodiment;
Fig. 17 is an illustration showing the state in which the main sheet body of the interlabial pad according to the embodiment is bent along the bending induction line, which is applied on the absorber;
Fig. 18 is a longitudinal cross section for describing that there is a protruded area, which projects towards the body side, provided in the sub-sheet body of the interlabial pad according to the embodiment;
Fig. 19 is a longitudinal cross section for describing the unbonded area between the fitted main sheet body and the sub-sheet body in a state where the center line of the labia coincide with the center line of the interlabial pad according to the embodiment;

Fig. 20 is a perspective view showing the wearing state of the interlabial pad according to the embodiment;

Fig. 21 is a cross section view showing the wearing state of the interlabial pad with a ready-made projection, which is fitted in a state where the center line of the labia and the center line of the interlabial pad are shifted from each other;

Fig. 22 is a cross section view showing the wearing state of the interlabial pad according to the embodiment, which is worn in a state where the center line of the labia and the center line of the interlabial pad according to the embodiment are shifted from each other;

Fig. 23 is a perspective view of the interlabial pad according to the embodiment, which is fitted in a state where the center line of the labia coincides with the center line of the interlabial pad according to the embodiment; and

Fig. 24 is a perspective view of the interlabial pad according to the embodiment, which is fitted in a state where the center line of the labia and the center line of the interlabial pad according to the embodiment are shifted from each other,

Fig. 25 is an illustration showing the state of experiment on measurement of separation force of an adhesive;

Fig. 26 is an illustration showing the state of experiment on measurement of shearing force of the adhesive;

Fig. 27 is an illustration showing the state where the interlabial pad according to the embodiment is used together with a sanitary napkin;

Fig. 28 is an illustration showing the state where the interlabial pad according to the embodiment is wrapped individually;

Fig. 29 is an illustration showing an example of a sanitary napkin of the related art having a ready-made projection;

Fig. 30 is an illustration showing an example of a incontinence support pad of the related art having a finger hole; and

Fig. 31 is an illustration for describing an example of the state of finger insert in the incontinence support pad of the related art having the finger hole.

Best Mode for Carrying Out the Invention

Now, the interlabial pad according to embodiments will be described by referring to the accompanying drawings.

[Basic Structure]

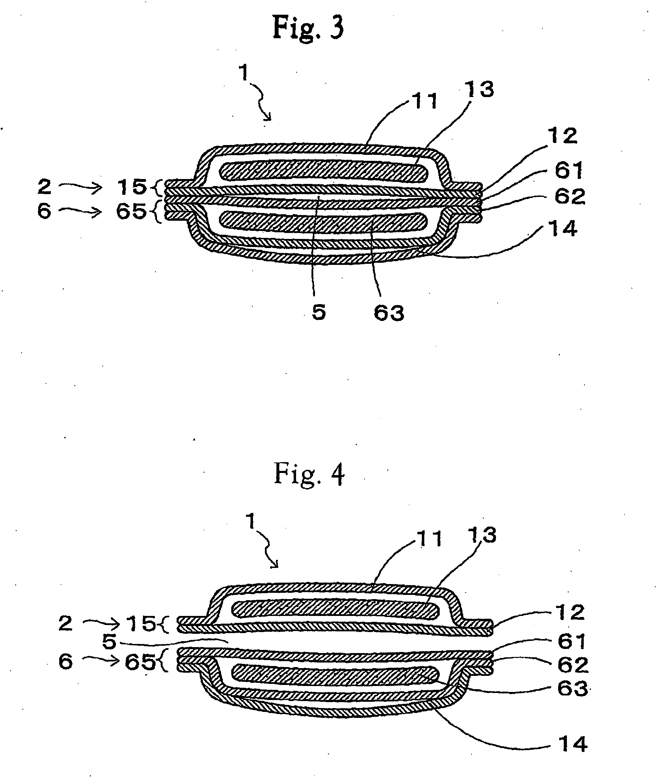

First, the configuration of an interlabial pad 1 according to an embodiment will be described. The interlabial pad 1 according to the embodiment has a main sheet body 2, a sub-sheet body 6, a mini sheet piece 14 attached on the sub-sheet body 6 on the opposite side to the body side. Fig. 1 is a schematic perspective view showing the body side of the interlabial pad 1 according to the embodiment, and Fig. 2 is a schematic perspective view showing the opposite side to the body of the interlabial pad 1 according to the embodiment. Fig. 3 is a cross section view of the interlabial pad according to the embodiment taken along the line X-X in Fig. 1, and Fig. 4 is a cross section of the interlabial pad according to the embodiment taken along the line Y-Y in Fig. 1.

An interlabial pad 1 according to the embodiment, as shown in Fig. 1, comprises a main sheet body 2 facing the body side and a sub-sheet body 6 facing the opposite side to the body. The main sheet body 2, as shown in Fig. 3 and Fig. 4, is formed as one body by bonding a surface side sheet 11 and a back side sheet 12 in a peripheral edge 15 so that an absorber 13 is sealed inside. It is preferable for the absorber 13 not to be sandwiched in the peripheral edge 15 where the sheets are bonded. For example, by bonding only the surface side sheet 11 and the back side sheet 12 and sealing the absorber 13 in the closed area in the peripheral edge 15, hardening of the peripheral edge 15 caused by sandwiching the absorber 13 in the peripheral edge 15 can be avoided. Thereby, more preferable wear felling can be achieved. The longitudinal dimension of the absorber 13 may be about that of the interlabial pad 1 or, in order for the absorber 13 not to be sandwiched in the above-described peripheral edge 15, the beforehand reduced dimension may be provided so as to be able to provide a gap of 2 to 10 mm apart from the contour of the interlabial pad 1.

The above-described sub-sheet body 6, as shown in Fig. 3 and Fig. 4, as in the main sheet body 2, is formed as one body by bonding a surface side sheet 61 and a back side sheet 62 in a peripheral edge 65 so that an absorber 63 is sealed inside. It is preferable for the absorber 63 not to be sandwiched in the peripheral edge 65 where the sheets are bonded.

The bonding of the surface side sheet 11 and the back side sheet 12 in the main sheet body 2 and that of the surface side sheet 61 and the back side sheet 62 in the sub-sheet body 6 are multiplied by a heat embossing adhesive and / or hot melting adhesive. Also, the absorber 13 is pasted to each of the surface side sheet 11 and the back side sheet 12 to prevent the interlayer separation therefrom, while the absorber 63 is pasted to each of the surface side sheet 61 and the back side sheet 62 to prevent the interlayer separation therefrom.

Bonding of the main sheet body 2 and the sub-sheet body 6 is not specifically limited, however, a heat embossing adhesive is more suitable than a hot melt adhesive in order to prevent the upper and lower layers from separating in wet state. In the bonding area of the main sheet body 2 and the sub-sheet body 6, only the peripheral edge of each sheet is bonded and the inside is unbonded (not pasted).

[Bonding of the Main Sheet Body and the Sub-Sheet Body]

As shown in Fig. 3, in the main sheet body 2 and the sub-sheet body 6, the peripheral edge 15 and the peripheral edge 65 are bonded in the front end and the back end. However, the Inside is left unbonded thereby forming a hollow part 5 therein. Also, as shown in Fig. 4, the back side sheet 12 of the main sheet body 2 and the surface side sheet 61 of the sub-sheet body 6 are unbonded thereby forming a hollow part 5 therein. Therefore, both of the sheet bodies 2 and 6 can be separately shifted in the vertical direction so that the main sheet 2 alone can be inserted between the labia of a wearer.

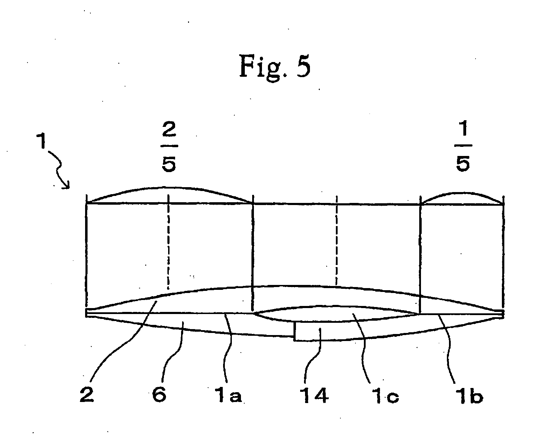

The right and left hand side of the main sheet body 2 and the sub-sheet body 6, as shown in Fig. 5, are bonded in a bonding area 1a in a range from the front end to 2/5 and in a bonding area 1b in a range from the back end to 1/5. The other area forms an unbonded area 1 c. As described, from the inner edge to the inside is left unbonded so that the unbonded area 1c, that is, in the view along the line Y-Y in Fig. 1, as shown in Fig. 4, the above-described hollow part 5 has a tunnel-like shape in the lateral direction. Therefore, in the range corresponding to the unbonded area 1c, the main sheet body 2 and the sub-sheet body 6 can be deformed to the right and left hand side.

[Mini Sheet Piece]

Next, a mini sheet piece 14 forming a finger insertion opening 19, which is attached to the opposite side to the body of the sub-sheet body 6 will be described. In the embodiment, as shown in Fig. 2, the mini sheet piece 14 is joined with the back side sheet 62 in a bonding area 17 in the outer edge of the back side sheet 62, and from the outer edge to the inside is left unbonded. Hence, the mini sheet piece 14 is attached over the area from one side of the back side sheet 62 to the other side thereby forming a hollow part 7 over the area from one side to the other side. The back side sheet 62 of the sub-sheet body 6 and the mini sheet piece 14 are bonded by the bonding area 17 in the peripheral edge 65 except for one of the wing 14a of the mini sheet piece 14 and the inside from the peripheral edge 65 is unbonded. Therefore, a finger insertion opening 19 capable of inserting a finger and a hollow part 7 to be a finger insert gap continued therefrom are formed between the opposite side face to the body 62a of the back side sheet 62 and the mini sheet piece 14. It is possible to provide a plurality of mini sheet pieces 14. In this case, the number of the bonding area 17 on the right and left hand sides increases on the back side sheet 62 in the longitudinal direction according to the number of the pieces. For example, if there are two mini sheet pieces 14, two bonding areas are to be provided on each side.

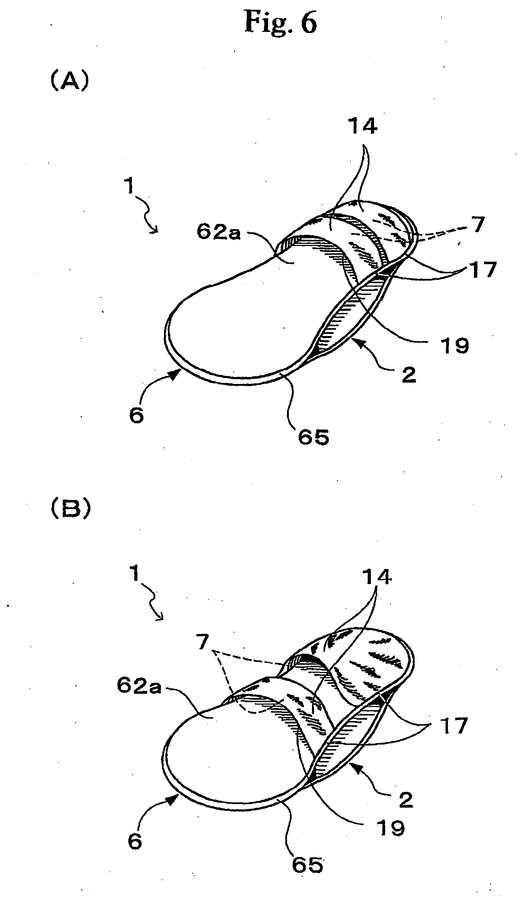

As described, in the case where a plurality of the mini sheet pieces 14 are provided, as shown in Fig. 6 (A), it is not necessary for all the mini sheet pieces 14 to be in the same shape and, for example, as shown in Fig. 6(B), each of the mini sheet pieces 14 may be in a different shape. Thereby, it can prevent menstrual blood from sticking to the finger and the amount of materials used for the mini sheet piece 14 can be decreased.

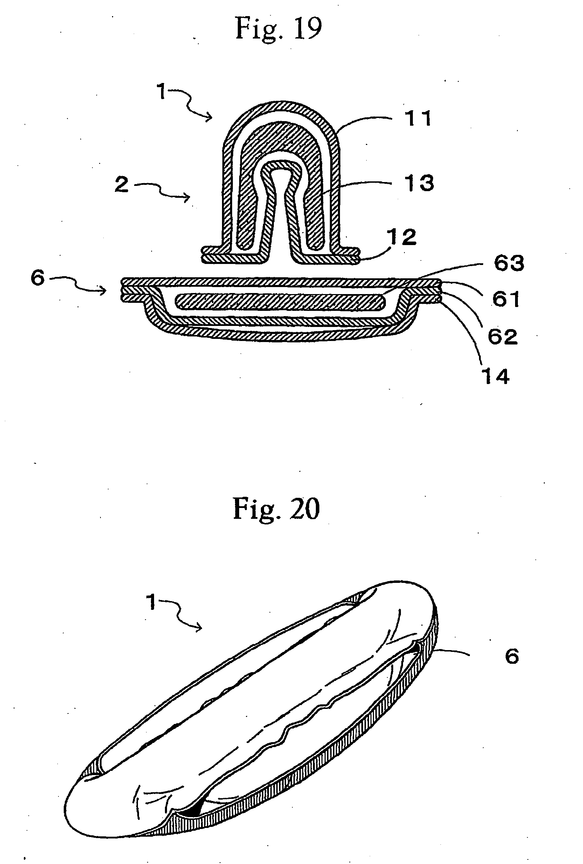

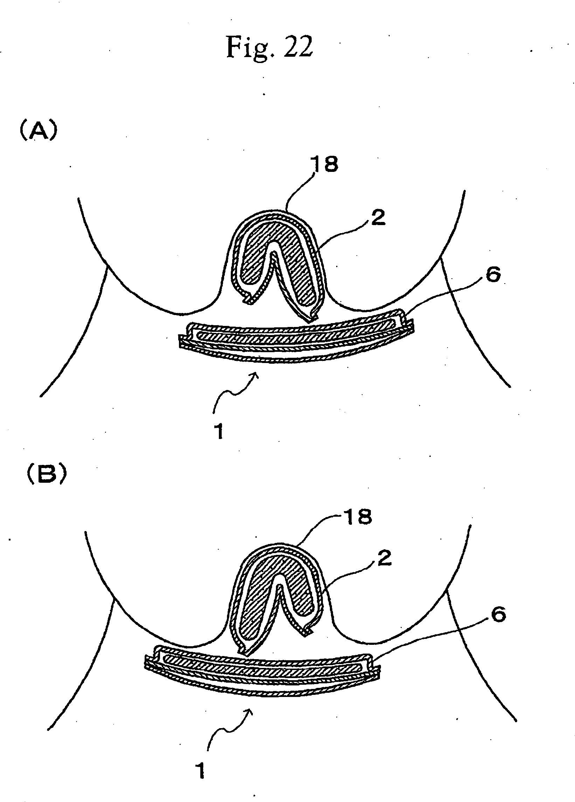

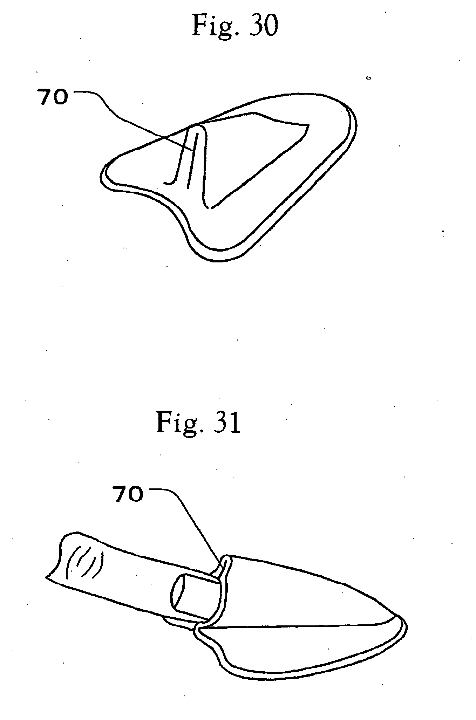

In the interlabial pad 1 according to the embodiment, the mini sheet piece 14 is attached so that the finger insertion opening 19 has an aperture large enough for the fingerbreadth in the direction of spread of the finger nail. Thereby, the flat-shaped finger tip is inserted naturally to come into contact with the sheet surface without being in the opposite direction to the sheet surface. In this respect, compared to an incontinence support pad of the related art (JP-A- 1994-506368), the complication of finger insert is remarkably improved to be simple. In other words, in the incontinence support pad of the related art, a finger hole 70 (numeral 76 in the above-described Publication) is closed (refer to Fig. 30, which corresponds to Fig. 20 in the above-described Publication) in the normal state. Therefore, first, a wearer inserts a finger in the direction making a right angle to the incontinence support pad (refer to Fig. 31,which corresponds to Fig. 22 in the above-described Publication) and then turns the finger. It is only then when the ball of the finger can be faced to the incontinence support pad side. On the contrary, in the interlabial pad 1 according to the embodiment, unlike the incontinence support pad of the related art in which the aperture of the fingerbreadth is secondarily formed in the direction of the surface of the back side sheet, the finger insertion opening 19 which is suitable for finger insert is primarily formed so as to be able to insert the finger naturally. Consequently, the wearer of the interlabial pad 1 can specify the direction of the finger insert. As a result, the ball of finger naturally detects the wearing location so that precise wearing of the pad between the labia can be more easily achieved.

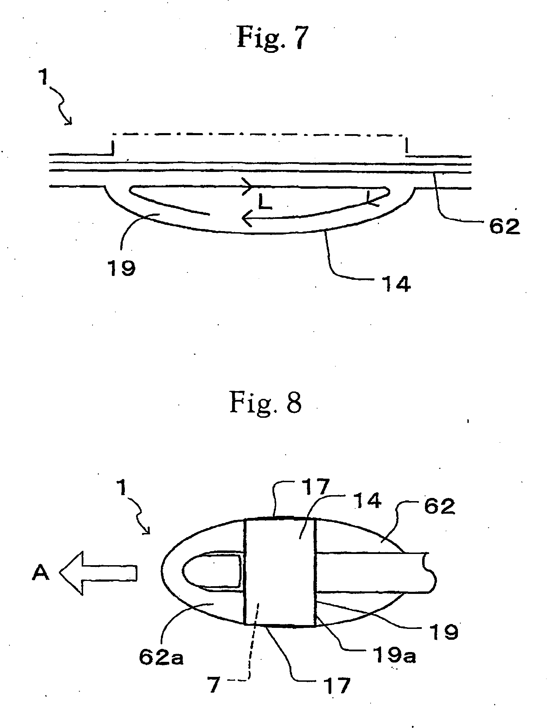

Fig. 7 is a cross section view of a part of the interlabial pad 1 in the lateral direction taken out from a part of the interlabial pad 1 for specifically describing "the whole girth inside the finger insertion opening 19". In Fig. 7, the part which is unnecessary for describing the length of "the whole girth inside the finger insertion opening 19" is shown by a dotted line. "The whole girth inside the finger insertion opening 19" is a distance denoted by "L" in Fig. 7.

The whole girth inside the above-described finger insertion opening 19 is preferable to be 30 to 120 mm, and more preferable to be 40 to 80 mm. When the whole girth inside the above-described second finger insertion opening 19 is shorter than 30 mm, the finger insertion opening 19 itself becomes small causing a difficulty in putting the finger in and out. On the other hand, when it is longer than 120 mm, the interlabial pad 1 can not be fixed to the finger. Therefore, it becomes hard for the ball of finger to be surely in contact with the sheet surface, which causes a problem when wearing. Accordingly, the length "L" in the embodiment is about 40 mm.

The whole girth inside the above-described finger insertion opening 19 is preferable to be 30 to 120 mm, and more preferable to be 40 to 80 mm. When the whole girth inside the above-described finger insertion opening 19 is shorter than 30 mm, the finger insertion opening 19 itself becomes small causing a difficulty in putting the finger in and out. On the other hand, when it is longer than 120 mm, the interlabial pad 1 can not be fixed to the finger. Therefore, it becomes hard for the ball of finger to be surely in contact with the sheet surface, which causes a problem when wearing. Accordingly, the length "L" in the embodiment is about 40 mm.

In the embodiment, as shown in Fig. 2, the unbonded part of the mini sheet piece 14 and the back side sheet 62 is only one of the wing 14a which forms the finger insertion opening 19. However, the other wing 14b can be also left unbonded. In this case, the length of the mini sheet piece 14 is preferable to be 10 % or more in the longitudinal direction, more preferable to be 10 to 80 %, and most preferable to be 30 to 60 %. Fig. 8 is an illustration showing the state where the mini sheet piece 14 has a length of 10 % or more in the longitudinal direction. As shown in the figure, it is clear that the direction of finger insert can be provided in "A" direction by the mini sheet piece 14 having the length as described. In regards to this, "the 10 % or more length of the mini sheet piece 14" serves to indicate the direction of the finger insert in the interlabial pad 1 according to the present invention. By having the length as described, there is no chance for the finger once being inserted to the finger insertion opening 19 to be slipped out therefrom or for the finger to be wandered inside the hollow part 7. Therefore, the ball of finger can be kept facing the sheet surface of the back side sheet 62.

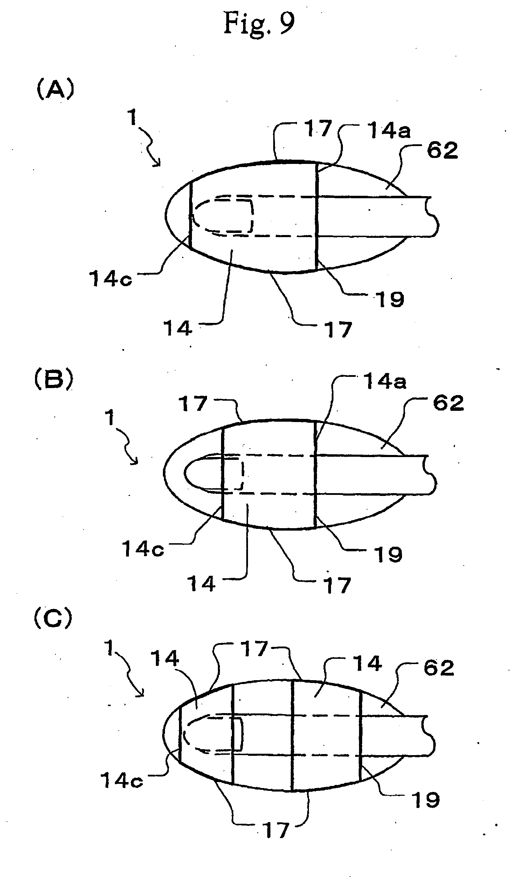

Fig. 9 is an illustration showing the position of the unbonded part in the back side sheet 62 of the mini sheet piece 14 attached on the interlabial pad 1 according to the present invention. In the case where a second unbonded part 14c is provided in addition to the unbonded part 14a forming the finger insertion opening 19, the tip of the finger may be exposed therefrom and there may be a contact with menstrual blood when wearing the interlabial pad 1. In this respect, as shown in Fig. 9(A), by providing the second unbonded part 14c in the position where the finger tip of the wearer is entirely covered, the finger can be kept unexposed so as to be sanitary as shown in Fig. 9(B).

Furthermore, as shown in Fig. 9(C), even in the case with a plurality of mini sheet pieces 14 are attached and there are a plurality of the unbonded parts, exposure of the finger can be prevented in the same manner if the second unbonded part 14c is provided in the endmost.

[Bonding Position of the Mini Sheet Piece]

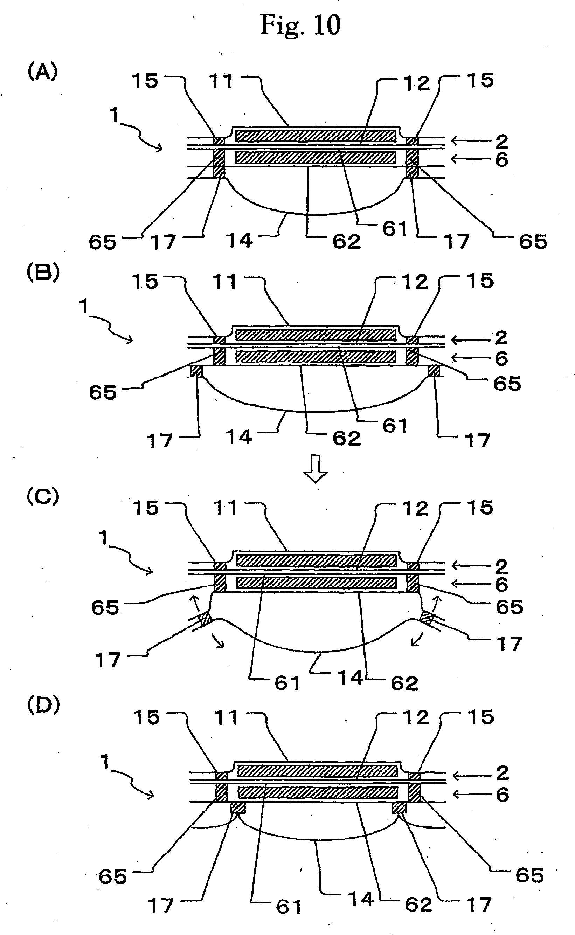

Next, the bonding condition of the main sheet body 2, the sub-sheet body 6, and the mini sheet piece 14 according to the embodiment will be described. As shown in Fig. 10(A), when the bonding area 17 between the mini sheet piece 14 and the back side sheet 62 is fixed together in the same position where the peripheral edge 15 as the bonding area between the surface side sheet 11 and the back side sheet 12 of the main sheet body 2, and the peripheral edge 65 as the bonding area between the surface side sheet 61 and the back side sheet 62 of the sub-sheet body 6, the outer edge side portion of the interlabial pad 1 becomes hard thereby influencing the wear feeling. This can be avoided by fixing the mini sheet piece 14 through positioning the bonding area 17 in the area other than the peripheral edge 65.

However, as shown in Fig. 10(B), when the bonding area 17 is positioned more outside of the peripheral edge 65 than the peripheral edge 65, as shown in Fig. 10(C), friction is generated by a movement in accordance with the action of the wearer so that it is considered to be possible that the wearer may be irritated.

As described, when positioning, as shown in Fig. 10(D), it is preferable that the peripheral edge 15 and the bonding area 17 are positioned shifted from each other and the bonding area 17 is positioned inner side than the peripheral edge 65.

When attaching the mini sheet piece 14, pressure sensitive hot melt, thermal sensitive hot melt and the like can be used as the adhesive and can be applied on the whole surface, or in line, spiral, dots and the like.

The mini sheet piece 14 may be cut beforehand so as to fit with the attaching part as described. Also, if the bonding area is positioned in a different position from other sheets, it may be cut together with other sheets.

[Shape of the Interlabial pad]

The shape of the interlabial pad according to the embodiment may be in any shapes such as elliptic-, ovoid-, gourd-, or drop-shape as long as it is suitable to be worn between the labia. However, with the same shape as in the embodiment, it can be provided to easily fit for both the labia and the finger.

Each sheet of the interlabial pad 1 according to the embodiment is made of a flexible sheet so that freely and elastically deformed by the external pressure. Hence, the shape of the interlabial pad 1 may be deformed on the appearance such as being bent or twisted, however, the above-described shape is achieved by clearing the deformation in each sheet and stretching it.

[Basic Material]

The material used for the surface side sheet 11 of the main sheet body 2 and the surface side sheet 61 of the sub-sheet body 6 are permeable material, and not specifically limited as long as it has the structure which permeates a liquid, such as a fabric, nonwoven fabric or perforated plastic sheet. In addition to a perforated film obtained by performing perforation, heat embossing, machine processing or the like on a thermoplastic film, a composite sheet of the perforated film and the nonwoven fabric, the materials shown below can be also used.

As the fabric and nonwoven fabric, examples of the natural fibers are cotton, silk, and hemp, examples of the regenerated fibers are regenerated cellulose fiber such as rayon fiber and acetate fiber, and examples of synthetic fibers are composite fibers of a single fiber and myelinated structure such as polyolefin fiber, polyacrylonitrile fiber, polyester fiber, polyamide fiber, polyvinyl alcohol fiber, polyurethane fiber, and nylon. Especially for the nonwoven fabrics, web forming can be performed either by dry method (carding, spun bonding, melt-blown, air-laid and the like) or wet method, or a plurality of the methods may be combined to be used. Examples of bonding methods are spun lacing using columnar water flow, thermal bonding, and needle punching.

Among the materials, considering the liquid mobility from the inner face of the labia, chemical stimulation by an activator, and adhesion with the inner wall of the labia, it is preferable to laminate rayon with 1.1 to 4.4 dtex fineness and 7 to 51 mm fiber length by 40 to 80 % of a total specific weight per unit area on the body surface side, and to laminate a mixture of rayon with 1.1 to 4.4 dtex fineness and 7 to 51 mm fiber length by 14 to 42 % of a total specific weight per unit area and PET with 1.1 to 4.4 dtex fineness and 7 to 51 mm fiber length by 6 to 18 % of a total specific weight per unit area on the clothing surface side. After laminating them so that the total specific weight per unit area of the two layers becomes 20 to 60 $g/m^2$, the fibers are entangled by water-flow interlacing treatment and then dried to prepare spun lace nonwoven fabric with the thickness of 0.13 to 0.50 mm. The spun lace nonwoven prepared as described is preferable. At this time, by mixing PET on the garment side , bulkiness can be easily maintained even if the permeable sheet becomes wet. Therefore, adhesion between the inner wall of the labia can be maintained.

Examples of a perforated plastic sheet which can be used are an air sheet of thermoplastic resin such as polyethylene (PE), polypropylene (PP), and polyethylene terephthalate (PET), and a perforated foamed material. Also about these, it is preferable to use it by making it milky by mixing a filler made of titanium oxide, calcium carbonate and the like within the range of 0.5 to 10 weight % if necessary. A perforated film obtained by forming perforation, thermal embossing, machine processing or the like on a thermoplastic film may be used. Furthermore, a composite sheet of the perforated film and nonwoven fabric may be used.

The material used for the absorber 13 and the absorber 63 may be any material as long as it is capable of absorbing and holding a body fluid (menstrual blood). However, it is preferable to use a single material or a mixture of the materials selected from the group comprising pulp, chemical pulp, rayon, acetate, cotton, particulate super absorbent polymer, super absorbent polymer fiber, and a composite fiber. Also, cellulose foam, a continuous foam and the like of synthetic resin can be also used as the absorber. Furthermore, an absorber obtained by grinding and molding the above-described sheet and the foam can be used. The method by which the materials are formed to be the absorber is not limited, however, the method such as air-laid, melt-blown, spun lacing, or paper-making method is employed for an absorber to be formed into a sheet to be used.

It is preferable for the absorber, although any material can be used as long as it is capable of absorbing and holding liquid (body fluid), to be bulky, hard-to-be deformed, less chemically stimulant, and highly flexible to fit into the labia. Specifically, a nonwoven sheet in which, 50 to 150 $g/m^2$ of pulp selected from the range of the fiber length of 1 to 10 mm is laminated on the garment face side and, on the body face side, 150 to 250 $g/m^2$ of a mixture obtained by mixing 60 to 90 % of rayon with 1.1 to 4.4dtex fineness and 20 to 51 mm fiber length with 40 to 10 % of natural cotton by this mixing ratio is laminated, which then to be formed into a sheet by dotted embossing to have 2 to 10 mm bulkiness, and more preferable to have 3 to 5 mm bulkiness. Thereby, body fluid can be easily transmitted from the body face side to the garment face side resulting in the improvement of the absorbing and holding capacity. Furthermore, by providing a mesh spun lace nonwoven fabric of rayon with 1.1 to 4.4dtex fineness and 25 to 51 mm fiber length by a specific weight per unit area of 15 to 40 $g/m^2$, the body fluid transmitted from the body face side can be dispersed by the mesh spun lace to be induced to almost all over the region of the pulp layer. Therefore, more body fluid can be effectively absorbed.

The material used for the back side sheet 12 of the main sheet body 2 and the back side sheet 62 of the sub-sheet body 6 are not specifically limited as long as it has a sheet-type structure such as a fabric, nonwoven fabric, or a plastic. However, examples of an impermeable material are an impermeable film mainly made of PE, PP or the like, a breathing resin film, and a material in which a breathing resin film is bonded to the back side of a nonwoven fabric such as a spun bond or spun lace on which water-repellent processing is performed. Considering the degree of softness by which the wear feeling is not influenced, for example, a film obtained by a specific weight per unit area of 15 to 30 $g/m^2$ mainly using LDPE (low density polyethylene) is used.

It is more preferable to reduce the contact ratio to decrease the friction drag value by embossing the above-described film to provide convex-shaped projections in order to, when the pad is worn between the labia, decrease the risk of the interlabial pad from being fallen off from the labia due to the high friction caused by the contact between the impermeable sheets, or with a pad used together, an underwear or the like.

By preparing the reveres side sheet 62 of the above-described sub-sheet body 6 using an impermeable material, menstrual blood held in the absorber 63 can be prevented from leaking out. Also, by preparing it using a wet permeable material, stuffiness can be decreased when wearing. Thereby, uncomfort felt by the wearers can be decreased when wearing.

It is preferable to select the material used for the mini sheet piece 14 considering the strength of the material so that it is not damaged when a finger is inserted. It is possible to select with no limitation a single material or the laminated material from the group comprising a nonwoven sheet, an elastic dilation nonwoven fabric, a film, a foam film, an elastic dilation film, a foam sheet, a tissue paper, and the like. Specifically a film of 15 to 30 $\mu$m thickness having an LDPE resin as the main component can be selected. Also, the mini sheet piece 14 can be prepared to have the tone of color, design, chroma which are different from those of the back side sheet 62 of the sub-sheet body 6 by coloring or printing a design or the like in order for the wearer to be able to easily discriminate the mini sheet piece 14.

As for the mini sheet piece 14, in order to effectively use the interlabial pad according to the present invention regardless of the finger tip size of the wearer, it is also effective to have the back side sheet 62 elastic or elastic-expanding characteristic in the lateral direction.

In order for the mini sheet piece 14 to have a stretching characteristic, a stretching spun bond nonwoven fabric can be used in which the stress is 0.1 to 0.5 N/25 mm at the time of 5 % stretching when being stretched at a constant speed by a stretching speed of 100 m/minute with a grip interval of 100 mm.

Also, in order for the mini sheet piece 14 to have an elastic dilation characteristic, a fiber sheet or film sheet using thermoplastic elastomer resin may be used. Also, the elastic dilation material such as the thermoplastic elastomer resin or natural rubber may be used alone or may be combined with a non-elastic dilation material to be used.

[Biodegradable Material, Water Soluble Material, Water Dispersible Material]

The interlabial pad 1 of the present invention can be formed of a biodegradable material, a water dispersible material, a water soluble material, or any combination of these materials. Thereby, the interlabial pad 1 after being used is to be naturally decomposed as time goes by or actively. Therefore, it can be flushed down to a toilet so that discard of the used interlabial pad 1 can be performed easily and cleanly. In other words, the wearer, when discarding the interlabial pad 1, simply goes to a toilet and open the leg towards the toilet bowl to drop the interlabial pad 1 into the toilet bowl. Hence, there is no need for the wearer to be bothered going through a complicated action such as discarding the used product using hands. In addition, there is an advantage that trashes left in the toilet can be decreased.

Furthermore, by preparing the wrapping container for individually wrapping the interlabial pad 1 according to the present invention by a biodegradable material and / or a water soluble material and / or water dispersible material, the wrapping container can be also flushed down to the toilet. Thereby, the wearer can be freed from the trouble of discarding the wrapping container and trashes in the toilet can be further decreased at the same time.

In this Specification, "biodegradablility" means that a substance is decomposed into gas such as carbon dioxide or methane, water, and biomass under anaerobic or aerobic condition according to the natural process under the existence of bacteria represented by actinomycetes and other microbes, and also means that the biodegradability (biodegradable rate and biodegradable degree) of the substance equals to a material naturally generated such as fallen leaves or a synthetic polymer generally.

"Water dispersible" has the same meaning as water degradable. It means a characteristic in which, while not influenced when used in a limited amount of moisture (menstrual blood), in a large amount of water or water current, the fabric is easily dispersed into small pieces at least to a degree where an ordinal toilet plumbing is not clogged. "Water soluble" is a characteristic in which, while not influenced when used in a limited amount of moisture (menstrual blood), the fabric is soluble in a large amount of water or water current.

The material is not specifically limited as long as it satisfies the above-mentioned conditions. However, the materials which can be used are shown below. First, a natural fiber and / or chemical fiber can be used for the fiber as a permeable material. Examples of the natural fiber are tissue, ground pulp, air laid pulp which is obtained by chemical-bonding a water soluble resin, and cotton. Examples of hydrophilic chemical fiber are rayon which is a regenerated cellulose, and feeble rayon, and examples of synthetic fiber are the one obtained by performing hydrophilic processing on polyester, polypropylene, polyethylene, ethylene vinyl acetate copolymer and the like. Also, examples of synthetic biodegradable fiber is poly lactate and polybutylene succinate, and examples of a water soluble material are carboximethyl cellulose, polyvinyl alcohol, and polyacrylonitrile. Especially, it is preferable to use the natural fiber such as pulp or cotton or biodegradable fiber such as rayon or poly lactate. It is also possible to use one of these materials alone or by mixing the materials by a predetermined combination to form a web or nonwoven fabric. Web forming of the synthetic degradable fabric such as poly lactate or polybuthylene succinate may be performed using a dry method, wet method or the like by carding, spun bonding, melt blow and air laid, or may be performed by a method in which a plurality of the methods are combined.

Examples of bonding are spun lace by columnar water flow, thermal bonding, needle punching, and chemical bonding. Example of method for forming a water dispersible fiber is a method of forming a water soluble paper in which a fiber is formed into a sheet by a hydrogen bonding of fibers, and a water soluble paper in which a fiber is formed into a sheet by entangling.

In order to keep an excellent water dispersiblity, it is preferable to have the fabric length within the range of 2 to 51 mm, and more preferable to have it within the range of 2 to 10 mm. Furthermore, if the strength is considered to be in a degree so that there is no damage generated when using the water dispersible fiber, it is desirable to select the fineness of the fiber (thickness) within the range of 1.1 to 4.4dtex.

Also, it is preferable to have a specific weight per unit area of 20 to 60 g/m$^2$. The break strength (the break strength when constant-stretching at grip interval of 100 mm and stretching speed at 100 mm/min) of the permeable material in both longitudinal and lateral direction are at least 800 mN/25 mm, and more preferable to be selected from the range of 1000 to 7000 mN/25 mm with the consideration of softness at the time of wearing.

A specific example of the permeable material is a wet spun lace nonwoven fabric prepared by mixing 5 to 10 mm of rayon fiber of 1. 1 to 4.4dtex and timber pulp at 90 : 10 to 70 : 30 weight ratio with a specific weight per unit area of 25 to 40 g/m$^2$ and the thickness of 0.2 to 0.5 mm. A plurality of pores may be provided on the permeable material. In this case, the pores may be formed to have a diameter within the range of 0.5 to 1.5 mm with the porous area ratio (rate of the porous area per unit area) within the range of 3 to 20 %.

A natural fiber and / or chemical fiber can be used for the absorber 13 and the absorber 63. Examples of the natural fiber are tissue, ground pulp, air laid pulp which is obtained by chemical bonding water soluble resin, and cotton. Examples of hydrophilic chemical fiber are rayon which is a regenerated cellulose, and feeble rayon, and examples of synthetic fiber are the one obtained by performing hydrophilic processing on polyester, polypropylene, polyethylene, ethylene vinyl acetate copolymer and the like. Also, examples of synthetic biodegradable fiber are poly lactate and polybutylene succinate, and examples of a water soluble material are carboximethyl cellulose, polyvinyl alcohol, and polyacrylonitrile. Especially, it is preferable to use the natural fiber such as pulp or cotton or biodegradable fiber such as rayon or poly lactate. It is also possible to use one of these materials alone or by mixing the materials by a predetermined combination. It is also possible to form a high absorbent polymer such as algin soda, amylum, starch, carboxymethyl cellulose into grains or fibers and mixing it with the high absorbent polymer and the like at a predetermined combination.

An example of the absorber 13 as described is a material prepared by enclosing timber pulp by laminating it with a specific weight per unit area of 150 to 500 g/m$^2$ to tissue and prepare it to the thickness of 2 to 10 mm by a pressing device. By mixing 5 to 30 g/m$^2$ of an absorbent such as starch with the above-mentioned absorber, it is possible to also improve the absorbance and holding ability of menstrual blood.

Examples of the impermeable material with biodegradability and / or water solubility are cellulose derivatives such as methyl cellulose, hydroxyethyl cellulose, carboxymethyl cellulose, water soluble polymer such as polyvinyl alcohol, algin soda, polyacrylic soda, polyacrylic ether, polyvinyl pyrrolidone, and a copolymer of isobutylene and maleic anhydride, poly lactic, polybutylene succinate, starch, and dextrin.

These materials may be used alone or mixed by a predetermined combination to be formed into a film sheet. Furthermore, repellent such as silicone may be applied or mixed thereto or it may be formed by a laminate processing performed on a nonwoven fabric.

A specific example of the sheet is a film obtained by preparing polyvinyl alcohol with a specific weight per unit area of 20 to 50 g/m$^2$ and to which 0.5 to 5 $\mu$m silicone or fluorine is applied at least on either side and, more preferably, on both sides.

Examples of the preferable material for the mini sheet piece 14 are polyvinyl alcohol film, and a laminated material of polyvinyl alcohol and tissue.

As the applicable bonding, boding by polyvinyl alcohol with water solubility or water dilatation characteristic, heat sealing, hydrogen bonding may be used alone or combined to be used as a bonding method.

Specific examples of the wrapping container made of a water soluble material or water dispersible material are a compound material obtained by laminating tissue with a specific weight per unit area of 15 to 40 g/m$^2$ and polyvinyl alcohol with a specific weight per unit area of 20 to 50 g/m$^2$ and applying silicone of 0.5 to 1 $\mu$m on the polyvinyl alcohol side, and a spun bond nonwoven fabric prepared with a specific weight per unit area of 15 to 40 g/m$^2$ mainly using poly lactic fiber.

[Size]

The length of the main sheet body 2 in the lateral direction is preferable to be 10 to 50 mm, and more preferable to be 20 to 30 mm. When the length in the lateral direction is longer than 50 mm, the periphery of the main sheet body 2 is rubbed against the femoral region of the wearer and the friction generated thereby exceeds the strength of labia itself so that the interlabial pad may fall off. Also, when the length in the lateral direction is shorter than 10 mm, the area of the main sheet body 2 which can be inserted between the labia becomes small thereby reducing the contact area with the inner face of the labia. Thereby, there generates a risk of the interlabial 1 pad being fallen off.

On the other hand, the length of the main sheet body 2 in the longitudinal direction is preferable to be 50 to 150 mm, and more preferable to be 80 to 120 mm. When the length in the longitudinal direction is longer than 150 mm, friction generated by the substantial flat area 4 which is not inserted between the labia being rubbed against the underwear or a sanitary napkin, and the strength of friction exceeds the strength of the labia itself so that the interlabial pad 1 may fall off. Also, when the length in the longitudinal direction is shorter than 50 mm, the range of the area of main sheet body 2 which can be inserted between the labia becomes small thereby reducing the contact area between the labia and the main sheet body 2. Thereby, there generates a risk of the interlabial pad 1 being fallen off.

The length of the sub-sheet body 6 in the lateral direction is preferable to be 10 to 60 mm, and more preferable to be 30 to 40 mm. When the length of the sub-sheet body 6 in the lateral direction is shorter than 60 mm, it becomes shorter than the length of the main sheet body 2 in the longitudinal direction, and to not be able to cover the pudenda so as to absorb menstrual blood leaked from the main sheet body 2. Therefore, the leak of menstrual blood may occur forward and backward.

By providing each sheet body in the length within the range as described, the interlabial pad 1 having an excellent prevention effect of menstrual blood leak with an excellent wear feeling can be achieved.

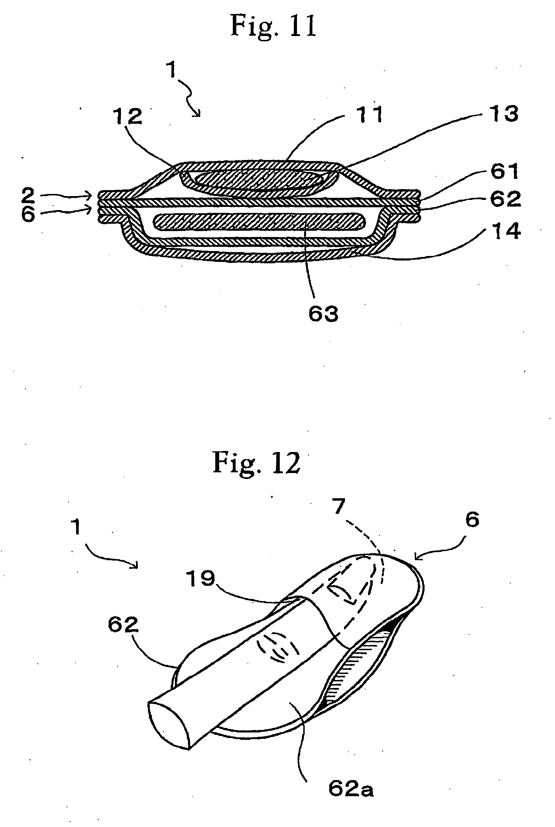

Incidentally, in the case where an impermeable material is used for the back side sheet 12 of the main sheet body 2, as shown in Fig. 11, the back side sheet 12 is provided to be the minimum size for covering the absorber 13, which is to be smaller than the surface side sheet 61 of the sub-sheet body 6. Thereby, the impermeable back side sheet 12 interrupts the menstrual blood which cannot be absorbed in the main sheet body 2 to be permeated to the sub-sheet body 6. As a result, it can prevent menstrual blood from leaking out of the interlabial pad 1.

[Finger insertion opening]

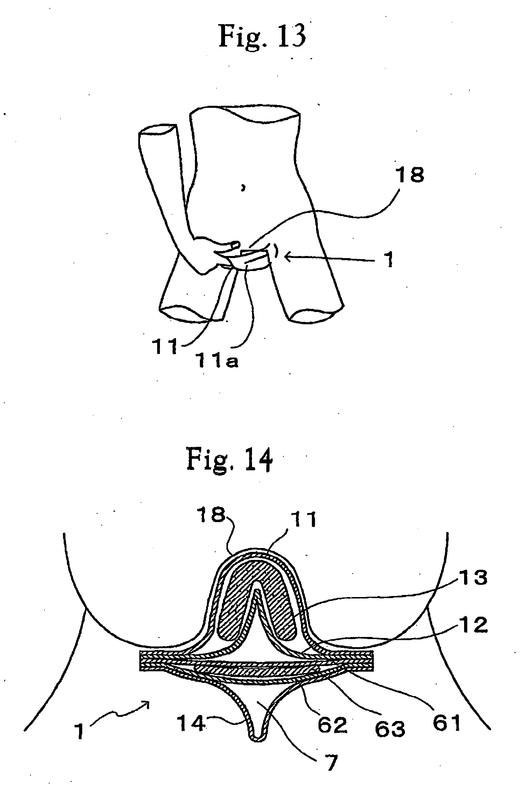

Next, the finger insertion opening 19, the finger insert gap 7 continued therefrom provided in the interlabial pad 1 according to the embodiment will be described. Fig. 12 is an explanatory illustration for describing the manner in which the finger is put in and out from the first finger insertion opening 19 to the hollow part 7 when using the interlabial pad 1 according to the embodiment. Fig. 13 is an illustration showing the state of wearing the interlabial pad according to the embodiment between the labia.

As shown in Fig. 12, the wearer inserts the finger from the finger insertion opening 19 to the hollow part 7 having its fingerprint side face being in contact with the opposite side surface to body 62a of the back side sheet 62 in the main sheet body 2. As described, by inserting the finger to the finger insertion opening 19, the fingerprint side surface of the first joint of the first finger, where many receptors exist, comes to be in contact with the opposite side surface to the body 62a of the back side sheet 62. Therefore, when leading the interlabial pad 1 to the labia, as shown in Fig. 13, the body side face 11 a of the surface side sheet 11 in the main sheet body 2 is provided to be in contact with labia 18 and, while detecting the concave and convex of the labia 18 by the finger tip with a keen sense, the interlabial pad 1 can be surely worn deep into between the labia 18 which is hard to be viewed.

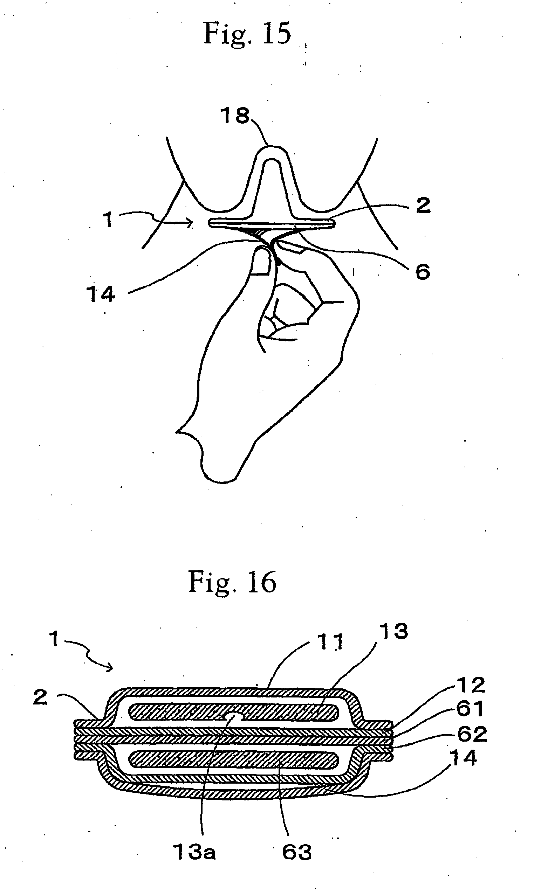

Also, inside of the mini sheet piece 14 forming the finger insert gap (the hollow part 7) is not pasted to the back side sheet 62. Therefore, when the finger is pulled out from the finger insertion opening 19 after wearing the interlabial pad 1, as shown in Fig. 14, the mini sheet piece 14 is loosened in the opposite direction to the body side. Hence, when removing the used interlabial pad 1, the loosened mini sheet piece 14 can be pulled out as shown in Fig. 15. Furthermore, by providing the mini sheet piece 14 using an impermeable or wet permeable material, the interlabial pad 1 can be removed without contaminating the finger even if the wearer grabs the mini sheet piece 14.

By providing microscopic concaves and convexes in the area to be in contact with the ball of finger in the back side sheet 12 of the main sheet body 2 and the back side sheet 62 of the sub-sheet body 6, the area can be reduced where the ball side of the finger tip comes to be in contact with the back side sheet 12 and the back side sheet 62. As a result, it makes possible to suppress friction and stuck generated between the finger tip and the interlabial pad 1. In this case, there is no chance for the interlabial pad 1 to be worn in the position which is not the intention of the wearer, which may otherwise occur due to the influence of the state of the finger tip of the wearer, such as the wet environment. Also, the finger can be smoothly pulled out after wearing so that the position shift after wearing can be prevented.

[Stiffness]

In the interlabial pad 1 according to the embodiment, the stiffness of the main sheet body 2 $\leqq$ the stiffness of the sub-sheet body 6. Thus, when the interlabial pad comes to be in contact with the body, it can be easily bent to be deformed into a convex shape.

Especially, as for the main sheet body 2 including the absorber 13, by providing the stiffness $\leqq$ 1.5 mN, the main sheet 2 alone is inserted between the labia without deforming the original shape of the labia of the wearer.

Now, an example of valuation method of stiffness of each sheet will be described in detail. The valuation method according to the embodiment is performed using Gurley's Stiffness Tester (YASUDA SEIKI).

A test piece is obtained by cutting the center area of the interlabial pad product by the dimension of 25 mm in the longitudinal direction and 38 mm in the lateral direction and is set in a chuck. After setting each weight, the scale is checked at the time when the test piece leaves the rotation rod of a pendulum and the stiffness in the lateral direction is valuated by conversion. At this time, the test piece is set by adjusting the length in one end so that the other end at 6. 3 mm of 38 mm side in the lateral direction becomes the pendulum side. Thereby, the stiffness of 25 mm dimension in 38 mm dimension in the lateral direction and 25 mm dimension in the longitudinal direction can be accurately measured.

On each weight, an auxiliary weight is attached so that the scale is in between 3 to 6 when the test piece leaves the rotation rod of the pendulum. Then, after pushing the switch, the scale is checked when the test piece leaves the rotation rod of the pendulum. This is performed for both ends in the above-described lateral direction.

The stiffness is to be obtained by the conversion expression shown below. That is, the stiffness is the value obtained by multiplying each of the values (1) to (3): the mean value (1) obtained from the scale on the left-hand side of the test piece and the scale on the right-hand side; the value (2) obtained by adding the weight [g] of the weight if the hole position when applying the weight is at 1 inch, twice the value of the weight [g] if the scale is at 2 inch, four times the value of the weight [g] if the scale is at 4 inch, respectively, and then dividing it by 5; the value (3) obtained by squaring the substantial dimension in the lateral direction 25 mm = 1 inch and then dividing it by the dimension in the longitudinal direction 25 mm = 1 inch; and then multiplying the obtained value by 9.88.

Specifically, it is calculated by the following expression.

$$\text{Stiffness (mN)} = \{(\text{left} + \text{right}) / 2\} \times \{(① \times 1 + ② \times 2 + ③ \times 4) / 5\} \times \{L2[\text{inch}] / W[\text{inch}]\} \times 9.88 \times 0.009807$$

(where, "left" means the scale obtained from the left-hand side of the test piece and "right" means "the scale obtained from the right-hand side of the test piece", the hole position when applying the weight at 1 inch is ① [g], 2 inch is ② [g], 4 inch is ③ [g], "L" means 1 [inch] which is the substantial dimension in the lateral direction and "W" means 1 [inch] which is the dimension in the longitudinal direction.

[Bending Induction Line]

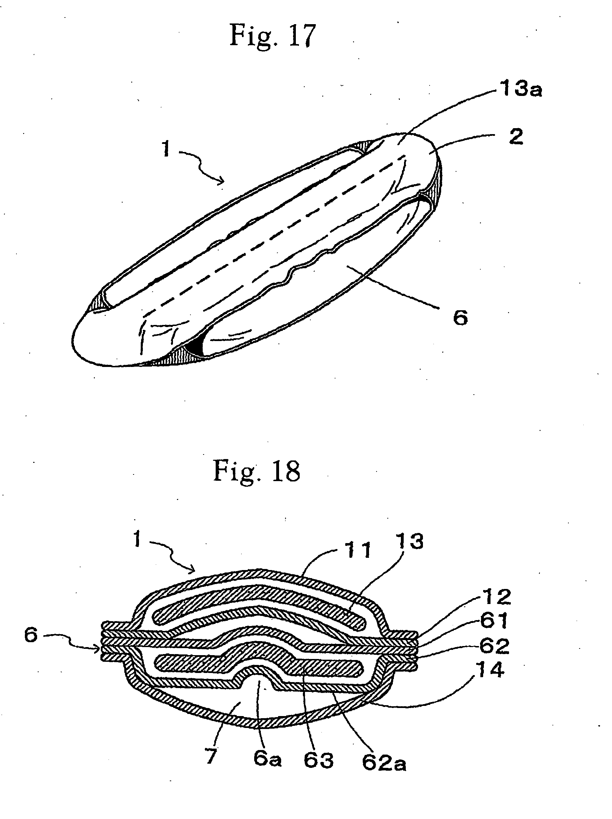

Next, a bending induction line applied to the absorber 13 which is included in the main sheet body 2 will be described. As shown in Fig. 16 which is a longitudinal cross section view of the interlabial pad 1 according to the embodiment, in the center area in the lateral direction of the absorber 13 included in the main sheet body 2, a bending induction line 13a is provided. Therefore, when a wearer wears the interlabial pad 1 according to the embodiment, as shown in Fig. 17, the main sheet body 2 can be easily bent towards the body side to be in a convex shape along the bending induction line 13a.

[Protruded Area]

Next, a protruded area 6a provided in the sub-sheet body 6 will be described. As shown in the longitudinal cross section view 18, the protruded area 6a is formed on the sub-sheet body 6 towards to body side. Therefore, when inserting a finger to a hollow 7, the wearer can insert the finger so that the ball of finger comes into contact with the opposite side face to the body 62a of the back side sheet 62 in the protruded area 6a. Thereby, the finger tip can be more like to be in one body with the interlabial pad 1. Thus, wearing of the interlabial pad 1 between the labia can be achieved more easily.

[Wearing State]

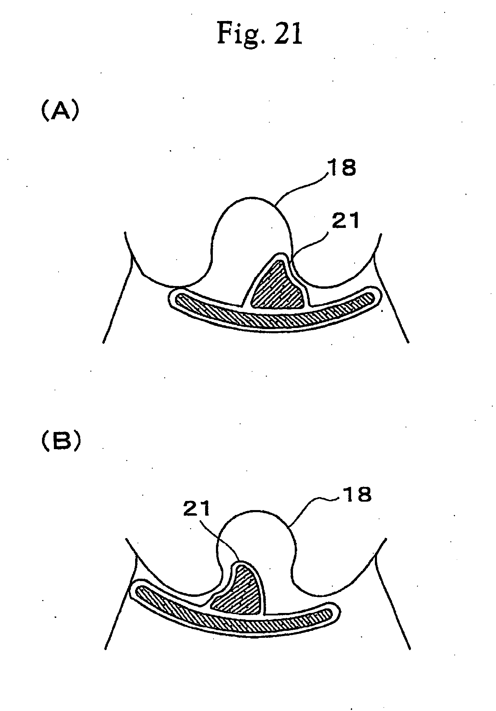

The state where the interlabial pad 1 according to the embodiment is fitted will be described. Fig. 19 is a longitudinal cross section view of an unbonded area showing the wearing state of the interlabial pad 1 according to the embodiment in a state where the center line of the labia coincides with the center line of the interlabial pad 1. Fig. 20 is a perspective view showing the wearing state of the interlabial pad 1 according to the embodiment. Fig. 21 is a cross section view showing the wearing state where there is a position shift in an interlabial pad with a ready-made projection, and Fig. 22 is a cross section view showing the wearing state where there is a position shift in the interlabial pad 1 according to the embodiment.

As shown in the above-described Fig. 5, in the interlabial pad 1 according to the embodiment, the unbonded area 1c is provided between the main sheet body 2 and the sub-sheet body 6. Therefore, in the unbonded area 1c, as shown in Fig. 19, the main sheet body 2 is deformed so as to form a convex shape towards the body side at the time of wearing. On the other hand, the sub-sheet body 6 stays substantially in the original shape without directly conforming to the deformation of the main sheet body 2. Subsequently, as shown in Fig. 20, the sub-sheet body 6 is deformed and positioned to cover the pudenda.

In the case where the center line of the interlabial pad is shifted either to the left-or right-hand side with respect to the center line of labia 18 of the wearer, if an interlabial pad with a ready-made projection 21 is used, as shown in Fig. 21, the ready-made projection 21 is positioned to be hang in the labia 18 of the wearer. Therefore, it becomes hard to tightly fit the projected area 21 between the labia of the wearer. As a result, there is a gap to be generated between the labia 18 of the wearer and the interlabial pad so that there may cause a menstrual blood leak.

On the contrary, with the interlabial pad 1 according to the embodiment, even if the center line of the interlabial pad 1 and the center line of the labia 18 are shifted either to the left-or right-hand side of the body of the wearer, as shown in Fig. 22, the main sheet body 2, which can be separated from the sub-sheet body 6, is deformed to a suitable shape to be inserted between the labia 18. Therefore, it can be inserted between the labia 18 in the longitudinal direction. Hence, there is no chance of a gap to be generated between the labia 18 and the interlabial pad 1 thereby no menstrual blood leak is likely to occur. Also, since the sub-sheet body 6 is not inserted between the labia 18, compared to the interlabial pad 1 with a single structure in which the whole thickness of the interlabial pad 1 is inserted between the labia 18, the foreign feeling felt by the wearer can be remarkably decreased.

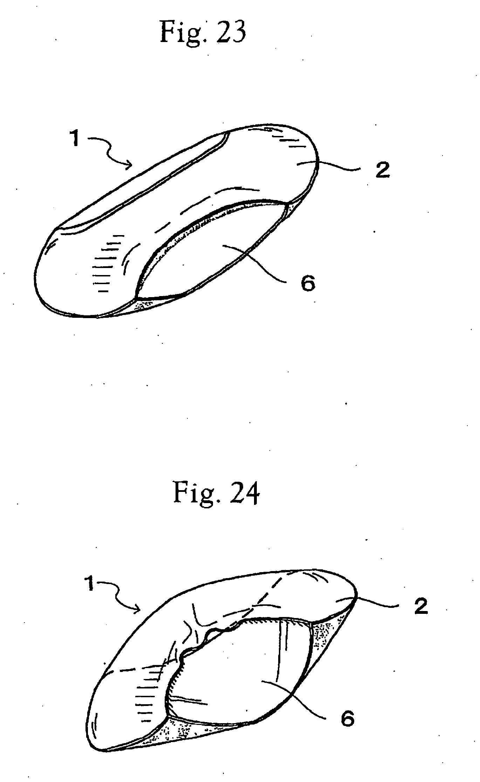

When viewing the state from the body side, where the interlabial pad 1 according to the embodiment is worn so that its center line coincides with the center line of the labia 18 of the wearer, the main sheet body 2 is deformed as shown in Fig. 23. On the contrary, when viewing the state from the body side, where the interlabial pad 1 according to the embodiment is fitted so that its center line is shifted from the center line of the labia 18 of the wearer, it is deformed as shown in Fig. 24.

[Application of Adhesive]

By applying an adhesive beforehand on a part of the surface side sheet 11 of the main sheet body 2 and/or the surface side sheet 61 of the sub-sheet body 6, the fixing and the adhesion of the interlabial pad 1 between the labia can be improved. Thereby, generation of a gap between the body and the interlabial pad 1 due to the body action of the wearer can be prevented. Thus, the risk of fall-off of the interlabial pad 1 can be decreased.

As the adhesive agent as described, a gel adhesive made of water-soluble polymer, a crosslinking agent, a flexibilizer and moisture can be used. Examples of the water soluble polymer used herein is gelatin, polyacrylic acid sodium, polyvinyl alcohol, and carboxymethyl cellulose. Examples of the crosslinking agent are water soluble metallic salt such as calcium chloride and magnesium sulfate, and examples of the flexibilizer are glycelol, wax, and paraffin.

As other adhesive agent, so-called a pressure sensitive hot melt can be also used. Incidentally, the pressure sensitive hot melt is mainly formed of synthetic rubber resin such as styrene-isoprene-styrene block copolymer (SIS), styrene-butadiene-styrene block copolymer (SBS), styrene-ethylene-butadiene-styrene block copolymer (SEBS), and styrene-ethylene-propylene-styrene block copolymer (SEPS). The pressure sensitive hot melt adhesive can be obtained by fused-mixing adhesion adder such as terpene resin or rosin resin and a flexibilizer such as wax to the pressure sensitive hot melt. Further, silicone adhesive agent can be used as other adhesive. An example of the silicone adhesive is a mixture obtained by mixing a crosslinking agent such as metallic salt of platinum, molybdenum, or antimony and a flexibilizer such as ester wax, glycerin, or machine oil.

If the application stability is taken into consideration, the pressure sensitive hot melt is preferable as adhesive agent. More specifically, it is an adhesive agent prepared by fused-mixing 15 to 25 weight % of SEBS, 15 to 35 weight % of flexibilizer, and 40 to 70 weight % of adhesive adder. Also, according to circumstances, it is possible to add antioxidant, antifluorescent or the like within the range of 0.1 to 1.0 weight % to the pressure sensitive hot melt.

It is preferable to cover the part where the adhesive is applied with a sheet which is obtained by coating silicon resin on a tissue paper, which is a generally obtainable separate paper, or a sheet obtained by coating silicon resin on a film. Thereby, damages or separation of the adhesive part can be prevented while being stored.

Examples of the manner in which the adhesive is applied are in the form of whole surface application or in dots, mesh, or lines. The application position of the adhesive agent is not specifically limited as long as it enables fixing of the pad to the body. However, specifically considering the existence of the pubic hair-grown part in the area in front of the labia, it is preferable to apply the adhesive near both end sides of the inter labia pad 1 in lines with about 1 to 5 mm width.

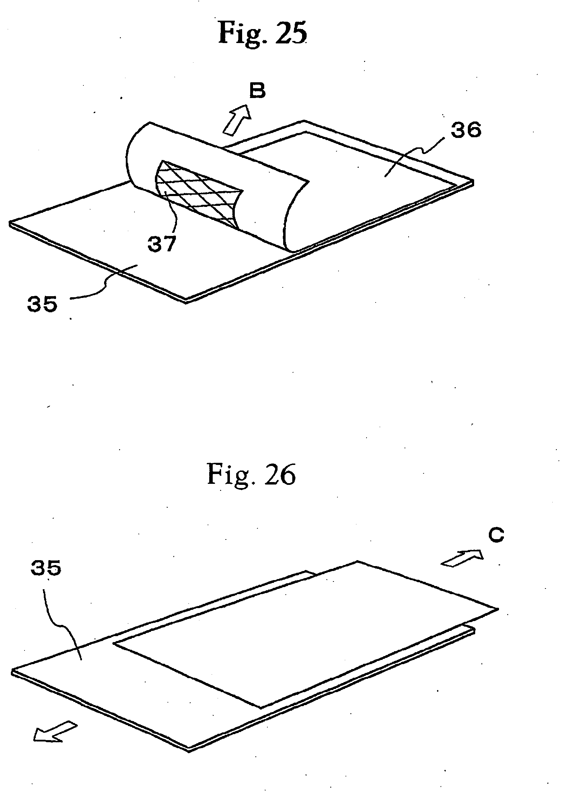

An example of valuation method of the adhesive strength will be described in detail. The valuation method is to measure the separation force (Fig. 25) and the shearing force of the adhesive (Fig. 26). A constant speed expansion tensile tester and a stainless plate of 80 mm x 50 mm is used therein.

As a preparation for the evaluation test, a test piece of a polyethylene film 36 in which an adhesive 37 applied within the range of 25 mm in width and 50 mm in length, is left for 30 minutes at a room temperature 20°C beforehand. Subsequently, the polyethylene film 72 is put lightly over a stainless plate 35 so that the adhesive 37 comes to be in contact with the stainless plate , and a 2 kg-roller is applied once (one way only). Then, it is left for 30 minutes at a room temperature (20°C) to fabricate a test piece.

The polyethylene film 36 part of the test piece which is fabricated as described is separated by being pulled in the arrow B direction shown in Fig. 25 for the separation force test, and is pulled in the arrow C direction shown in Fig. 26 for the shearing force test. The test condition is provided to be the chuck interval (grip interval) of 70 mm and testing speed at 100 mm/min.

In the case where the forces are measured by the measurement method described above, considering the burden imposed on the skin of the wearer, it is preferable that the measurement value of the separation force be 100 to 2000 mN/25 mm and that of the shearing force be 2900 to 15000 mN/25 mm.

[Other Applicable Embodiment of the Interlabial pad]

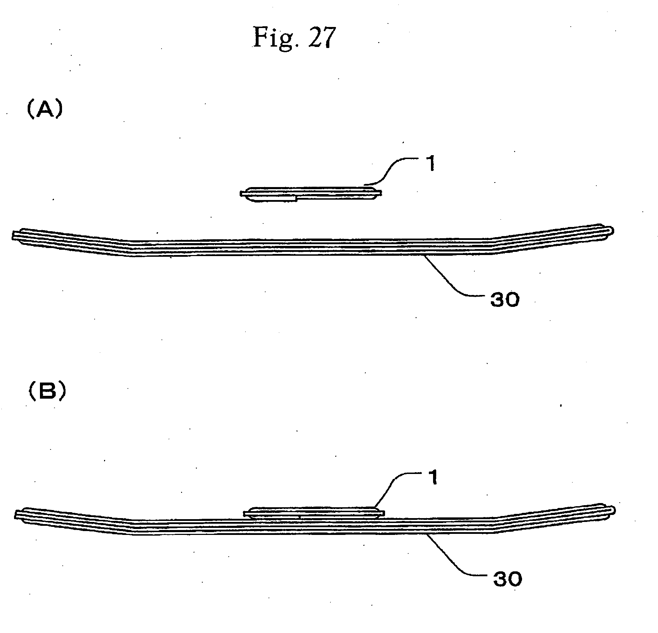

The interlabial pad 1 according to the embodiment, as shown in Fig. 27, can be used together with an ordinary sanitary napkin 30. As for the wearing method, the interlabial pad 1 is worn in between the labia and a sanitary napkin 30 is worn to the underwear. By using it together with a sanitary napkin as described, the Interlabial pad 1 of the present invention can be effectively used even on a occasion expecting a large quantity of menstrual blood.

[Individual Wrapping]

When individually wrapping the interlabial pad 1 according to the present invention, it is preferable to prepare the pad so that in order to be able to insert a finger right after opening the wrapping container, the pad may be wrapped anisotropic to the wrapping container so that the opening direction and the finger tip insert direction becomes the same, or the mini sheet piece 14 for finger insert can be positioned to be near the opening section of the wrapping containers.

It is also, as shown in Fig. 28, preferable to wrap the interlabial pad 1 by folding it in such manner that the finger insertion opening 19 is naturally opened when opening the wrapping container 40 so that the wearer can insert a finger to the finger insertion opening 19 of the interlabial pad 1 right after opening. Thereby, the wearer can easily recognize the position of finger insert. As a result, the Interlabial pad 1 can be worn more quickly and easily

Industrial Applicability

With the present invention as described, even in the case where the center line of the interlabial pad and the center line of the labia of the wearer are shifted either to the left-or right-hand side of the body of the wearer, the main sheet body 2 is suitably deformed to be inserted between the labia of the wearer thereby preventing menstrual blood leak in the lateral direction.

**Claims**

1.  An Interlabial pad (1) comprising a pair of absorbing sheet bodies (2, 6) for absorbing body fluid, each of the absorbing sheet bodies(2, 6) being unbonded at the right and left side edges relative to the longitudinal direction while being bonded at both ends in the longitudinal direction, wherein the unbonded areas in each of the right and left side edges relative to the longitudinal direction of the interlabial pad (1) are formed in a range of 2/5 to 4/5 of the total length of each side edge, and wherein the pair of absorbing sheet bodies (2, 6) comprise:

    a main sheet body (2) comprising a first permeable surface side sheet (11) facing a labia side, a first permeable or impermeable back side sheet (12) facing the opposite side to the labia side and a first absorber (13) for absorbing body fluid, wherein the first permeable surface side sheet (11) and the first back side sheet (12) are bonded to each other to enclose the first absorber (13); and

a sub-sheet body (6) comprising a second permeable surface side sheet (61) facing the labia side, a second impermeable back side sheet (62) facing a garment side and a second absorber (63) for absorbing body fluid, wherein the second permeable surface side sheet (61) and the second back side sheet (62) are bonded to each other to enclose the second absorber (63);

wherein the sub-sheet body (6) further includes a mini sheet piece (14) bonded to the right and left side edges of the second back side sheet (62) of the sub-sheet body (6) to form a finger insertion opening (19) between the second back side sheet (62) and the mini sheet piece (14) on the garment side, wherein the mini sheet piece (14) has a length which is from 10% to 80% of the total length of the interlabial pad (1) In the longitudinal direction, and wherein the width of the finger insertion opening (19) is between 30 mm and 120 mm.

2. The interlabial pad (1) according to claim 1, wherein the unbonded areas of the main sheet body (2) and the sub-sheet body (6) of the right and left side edges are symmetrical with respect to the centre line provided In the longitudinal direction of the interlabial pad (1).

3. The interlabial pad (1) as claimed in claim 1 or 2, wherein the width of the main sheet body (2) in the lateral direction is equal to, or is shorter than, that of the sub-sheet body (6) in the lateral direction.

4. The interlabial pad (1) as claimed in any one of claims 1 to 3, wherein the stiffness of the main sheet body (2) in the lateral direction is less than that of the sub-sheet body (6).

5. The interlabial pad (1) as claimed in claim 4, wherein the stiffness of the main sheet body (2) in the lateral direction Is not more than 1.5 mN.

6. The interlabial pad (1) as claimed in any one of claims 1 to 5, wherein the first absorber (13) inside the main sheet body (2) comprises a line for inducing bending in the centre of the lateral direction.

7. The interlabial pad (1) as claimed in any one of claims 1 to 6, wherein the sub-sheet body (6) includes a protruded area (6a), which protrudes towards the labia side.

8. The interlabial pad (1) as claimed in any one of claims 1 to 7, wherein the right and left side edges of the main sheet body and the sub-sheet body are bonded in a bonding area in a range from the front end to 2/5 and in a bonding area in a range from the back end to 1/5 and the other area forms the unbonded area.

9. The interlabial pad (1) according to any one of claims 1 to 8. wherein the interlabial pad (1) is used together with a sanitary napkin.

10. The interlabial pad (1) according to any one of claims 1 to 9, wherein the interlabial pad (1) is a pad for an incontinence of urine.

11. The Interlabial pad (1) according to any one of claims 1 to 9, wherein the interlabial pad (1) is a pad for absorbing vaginal discharge.

12. A wrapping body comprising;

the interlabial pad (1) as claimed in any one of claims 1 to 11; and
a wrapping container (40) for individually wrapping the interlabial pad(1);

wherein the interlabial pad (1) is enclosed in the wrapping container (40).

13. The wrapping body as claimed in claim 12 wherein the interlabial pad (1) is enclosed in the wrapping container (40) such that the finger insertion opening (19) formed by the mini sheet piece (14) is opened towards a wearer when opening the wrapping container (40).

**Patentansprüche**

1. Ein interlabiales Kissen (1), das ein Paar absorbierende Schichtkörper (2, 6) zum Absorbieren von Körperflüssigkeit beinhaltet, wobei jeder der absorbierenden Schichtkörper (2, 6) an dem rechten und dem linken Seitenrand relativ zu der longitudinalen Richtung unverbunden ist, während er an beiden Enden in der longitudinalen Richtung verbunden ist, wobei die unverbundenen Stellen an jedem von dem rechten und dem linken Seitenrand relativ zu der longitudinalen Richtung des interlabialen Kissens (1) in einem Bereich von 2/5 bis 4/5 der Gesamtlänge jedes Seitenrands gebildet sind, und wobei das Paar absorbierende Schichtkörper (2, 6) Folgendes beinhaltet:

    einen Hauptschichtkörper (2), der eine erste durchlässige Oberflächenseitenschicht (11), welche einer Labialseite zugewandt ist, eine erste durchlässige oder undurchlässige Hinterseitenschicht (12), welche der der Labialseite gegenüberliegenden Seite zugewandt ist, und einen ersten Absorber (13) zum Absorbieren von Körperflüssigkeit beinhaltet, wobei die erste durchlässige Oberflächenseitenschicht (11) und die erste Hinterseitenschicht (12) miteinander verbunden sind, um den ersten Absorber (13) einzuschließen; und

    einen Unterschichtkörper (6), der eine zweite durchlässige Oberflächenseitenschicht (61), welche der Labialseite zugewandt ist, eine zweite undurchlässige Hinterseitenschicht (62), welche einer Kleidungsseite zugewandt ist, und einen zweiten Absorber (63) zum Absorbieren von Körperflüssigkeit beinhaltet, wobei die zweite durchlässige Oberflächenseitenschicht (61) und die zweite Hinterseitenschicht (62) miteinander verbunden sind, um den zweiten Absorber (63) einzuschließen;

    wobei der Unterschichtkörper (6) ferner ein Minischichtstück (14) beinhaltet, das mit dem rechten und dem linken Seitenrand der zweiten Hinterseitenschicht (62) des Unterschichtkörpers (6) verbunden ist, um zwischen der zweiten Hinterseitenschicht (62) und dem Minischichtstück (14) auf der Kleidungsseite eine Fingereinführungsöffnung (19) zu bilden, wobei das Minischichtstück (14) eine Länge aufweist, die in der longitudinalen Richtung 10 % bis 80 % der Gesamtlänge des interlabialen Kissens (1) beträgt, und wobei die Breite der Fingereinführungsöffnung (19) zwischen 30 mm und 120 mm beträgt.

2. Interlabiales Kissen (1) gemäß Anspruch 1, wobei die unverbundenen Stellen des Hauptschichtkörpers (2) und des Unterschichtkörpers (6) des rechten und des linken Seitenrands mit Bezug auf die Mittellinie, die in der longitudinalen Richtung des interlabialen Kissens (1) bereitgestellt ist, symmetrisch sind.

3. Interlabiales Kissen (1) gemäß Anspruch 1 oder 2, wobei die Breite des Hauptschichtkörpers (2) in der lateralen Richtung gleich der des Unterschichtkörpers (6) in der lateralen Richtung oder kürzer als diese ist.

4. Interlabiales Kissen (1) gemäß einem der Ansprüche 1 bis 3, wobei die Steifigkeit des Hauptschichtkörpers (2) in der lateralen Richtung geringer als die des Unterschichtkörpers (6) ist.

5. Interlabiales Kissen (1) gemäß Anspruch 4, wobei die Steifigkeit des Hauptschichtkörpers (2) in der lateralen Richtung nicht mehr als 1,5 mN beträgt.

6. Interlabiales Kissen (1) gemäß einem der Ansprüche 1 bis 5, wobei der erste Absorber (13) innerhalb des Hauptschichtkörpers (2) in der Mitte der lateralen Richtung eine Linie zum Induzieren von Biegen beinhaltet.

7. Interlabiales Kissen (1) gemäß einem der Ansprüche 1 bis 6, wobei der Unterschichtkörper (6) eine hervorstehende Stelle (6a) umfasst, die zu der Labialseite hin hervorsteht.

8. Interlabiales Kissen (1) gemäß einem der Ansprüche 1 bis 7, wobei der rechte und der linke Seitenrand des Hauptschichtkörpers und des Unterschichtkörpers an einer Verbindungsstelle in einem Bereich von dem vorderen Ende bis zu 2/5 und an einer Verbindungsstelle in einem Bereich von dem hinteren Ende bis zu 1/5 verbunden sind und die andere Stelle die unverbundene Stelle bildet.

9. Interlabiales Kissen (1) gemäß einem der Ansprüche 1 bis 8, wobei das interlabiale Kissen (1) zusammen mit einer Damenbinde verwendet wird.

10. Interlabiales Kissen (1) gemäß einem der Ansprüche 1 bis 9, wobei das interlabiale Kissen (1) ein Kissen für eine Urininkontinenz ist.

11. Interlabiales Kissen (1) gemäß einem der Ansprüche 1 bis 9, wobei das interlabiale Kissen (1) ein Kissen zum Absorbieren von Scheidenausfluss ist.

**12.** Ein Verpackungskörper, der Folgendes beinhaltet:

das interlabiale Kissen (1) gemäß einem der Ansprüche 1 bis 11; und
einen Verpackungsbehälter (40) zum einzelnen Verpacken des interlabialen Kissens (1);

wobei das interlabiale Kissen (1) in dem Verpackungsbehälter (40) eingeschlossen ist.

**13.** Verpackungskörper gemäß Anspruch 12, wobei das interlabiale Kissen (1) so in dem Verpackungsbehälter (40) eingeschlossen ist, dass die Fingereinführungsöffnung (19), die von dem Minischichtstück (14) gebildet wird, zu einer Trägerin hin geöffnet ist, wenn der Verpackungsbehälter (40) geöffnet wird.

**Revendications**

**1.** Une protection interlabiale (1) comprenant une paire de corps formant feuillets absorbants (2, 6) destinés à absorber du fluide corporel, les corps formant feuillets absorbants (2, 6) n'étant ni l'un ni l'autre collés au niveau des bords de côtés droit et gauche par rapport à la direction longitudinale tout en étant collés au niveau des deux extrémités dans la direction longitudinale, où les zones non collées dans chacun des bords de côtés droit et gauche par rapport à la direction longitudinale de la protection interlabiale (1) sont formées sur une étendue allant des 2/5 aux 4/5 de la longueur totale de chaque bord de côté, et dans laquelle la paire de corps formant feuillets absorbants (2, 6) comprend :

un corps formant feuillet principal (2) comprenant un premier feuillet perméable côté surface (11) faisant face à un côté lèvre, un premier feuillet perméable ou imperméable côté dos (12) faisant face au côté opposé au côté lèvre et un premier élément absorbeur (13) destiné à absorber du fluide corporel, où le premier feuillet perméable côté surface (11) et le premier feuillet côté dos (12) sont collés l'un à l'autre pour enfermer le premier élément absorbeur (13) ; et
un corps formant sous-feuillet (6) comprenant un deuxième feuillet perméable côté surface (61) faisant face au côté lèvre, un deuxième feuillet imperméable côté dos (62) faisant face à un côté vêtement et un deuxième élément absorbeur (63) destiné à absorber du fluide corporel, où le deuxième feuillet perméable côté surface (61) et le deuxième feuillet côté dos (62) sont collés l'un à l'autre pour enfermer le deuxième élément absorbeur (63) ;

dans laquelle le corps formant sous-feuillet (6) inclut de plus un morceau formant mini-feuillet (14) collé sur les bords de côtés droit et gauche du deuxième feuillet côté dos (62) du corps formant sous-feuillet (6) pour former une ouverture d'insertion de doigt (19) entre le deuxième feuillet côté dos (62) et le morceau formant mini-feuillet (14) sur le côté vêtement, où le morceau formant mini-feuillet (14) présente une longueur qui fait de 10 % à 80 % de la longueur totale de la protection interlabiale (1) dans la direction longitudinale, et où la largeur de l'ouverture d'insertion de doigt (19) fait entre 30 mm et 120 mm.

**2.** La protection interlabiale (1) selon la revendication 1, dans laquelle les zones non collées du corps formant feuillet principal (2) et du corps formant sous-feuillet (6) des bords de côtés droit et gauche sont symétriques par rapport à la ligne centrale fournie dans la direction longitudinale de la protection interlabiale (1).

**3.** La protection interlabiale (1) telle que revendiquée dans la revendication 1 ou 2, dans laquelle la largeur du corps formant feuillet principal (2) dans la direction latérale est égale à, ou est plus courte que celle du corps formant sous-feuillet (6) dans la direction latérale.

**4.** La protection interlabiale (1) telle que revendiquée dans n'importe laquelle des revendications 1 à 3, dans laquelle la rigidité du corps formant feuillet principal (2) dans la direction latérale est inférieure à celle du corps formant sous-feuillet (6).

**5.** La protection interlabiale (1) telle que revendiquée dans la revendication 4, dans laquelle la rigidité du corps formant feuillet principal (2) dans la direction latérale n'est pas supérieure à 1,5 mN.

**6.** La protection interlabiale (1) telle que revendiquée dans n'importe laquelle des revendications 1 à 5, dans laquelle le premier élément absorbeur (13) à l'intérieur du corps formant feuillet principal (2) comprend une ligne destinée à susciter une flexion dans le centre de la direction latérale.

7.  La protection interlabiale (1) telle que revendiquée dans n'importe laquelle des revendications 1 à 6, dans laquelle le corps formant sous-feuillet (6) inclut une zone en saillie (6a), laquelle fait saillie en direction du côté lèvre.

8.  La protection interlabiale (1) telle que revendiquée dans n'importe laquelle des revendications 1 à 7, dans laquelle les bords de côtés droit et gauche du corps formant feuillet principal et du corps formant sous-feuillet sont collés dans une zone de collage sur une étendue allant de l'extrémité avant aux 2/5 et dans une zone de collage sur une étendue allant de l'extrémité arrière au 1/5 et l'autre zone forme la zone non collée.

9.  La protection interlabiale (1) selon n'importe laquelle des revendications 1 à 8, dans laquelle la protection interlabiale (1) est utilisée conjointement à une serviette sanitaire.

10. La protection interlabiale (1) selon n'importe laquelle des revendications 1 à 9, dans laquelle la protection interlabiale (1) est une protection destinée à une incontinence urinaire.

11. La protection interlabiale (1) selon n'importe laquelle des revendications 1 à 9, dans laquelle la protection interlabiale (1) est une protection destinée à absorber des pertes vaginales.

12. Un corps formant emballage comprenant :

    la protection interlabiale (1) telle que revendiquée dans n'importe laquelle des revendications 1 à 11 ; et
    un conteneur d'emballage (40) destiné à emballer individuellement la protection interlabiale (1) ;

    dans lequel la protection interlabiale (1) est enfermée dans le conteneur d'emballage (40).

13. Le corps formant emballage tel que revendiqué dans la revendication 12 dans lequel la protection interlabiale (1) est enfermée dans le conteneur d'emballage (40) de telle sorte que l'ouverture d'insertion de doigt (19) formée par le morceau formant mini-feuillet (14) est ouverte en direction d'une utilisatrice lors de l'ouverture du conteneur d'emballage (40).

# Fig. 1

# Fig. 2

## Fig. 3

## Fig. 4

Fig. 5

$\dfrac{2}{5}$

$\dfrac{1}{5}$

1

2　6　1a　14　1c　1b

# Fig. 6

(A)

(B)

## Fig. 7

## Fig. 8

## Fig. 9

(A)

(B)

(C)

# Fig. 10

(A)

(B)

(C)

(D)

# Fig. 11

# Fig. 12

EP 1 410 776 B1

Fig. 13

Fig. 14

29

Fig. 15

18

2

1

6

14

Fig. 16

1

2

11  13

12
61
62
14

13a   63

Fig. 17

Fig. 18

EP 1 410 776 B1

Fig. 19

Fig. 20

32

# Fig. 21

(A)

(B)

# Fig. 22

(A)

(B)

EP 1 410 776 B1

Fig. 23

Fig. 24

35

## Fig. 25

## Fig. 26

Fig. 27

(A)

1

30

(B)

1

30

Fig. 28

14

40

19

Fig. 29

Fig. 30

70

Fig. 31

70

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP HEI518523 B

- JP 6506368 A